# FASCICULE DE BREVET EUROPEEN

(11) **EP 3 541 921 B1**
(45) Date de publication et mention de la délivrance du brevet: **01.07.2020**
(21) Numéro de dépôt: 17801686.1
(22) Date de dépôt: 16.11.2017
(51) Int. Cl.: C12M 1/107, C12M 1/16, C12M 1/00, C12M 1/02

(54) **SYSTÈME DE PRODUCTION DE BIOGAZ À PARTIR DE BIOMASSE SOLIDE ET PROCÉDÉ DE BIOGAZ CORRESPONDANT**
SYSTEM ZUR HERSTELLUNG VON BIOGAS AUS FESTER BIOMASSE UND ZUGEHÖRIGES BIOGASVERFAHREN
SYSTEM FOR PRODUCING BIOGAS FROM SOLID BIOMASS AND CORRESPONDING BIOGAS METHOD

(30) Priorité: 17.11.2016 FR 1661168
(43) Date de publication de la demande: 25.09.2019
(73) Titulaire: YANNCO, 92800 Puteaux (FR)
(72) Inventeur: MERCIER, Yann, 92800 Puteaux (FR)
(74) Mandataire: Casalonga
(86) Numéro de dépôt international: PCT/EP2017/079499
(87) Numéro de publication internationale: WO 2018/091603

(56) Documents cités:
- EP-A2- 0 023 176
- BE-A- 427 335
- FR-A1- 2 500 256
- KR-A- 20130 020 444
- US-A- 5 958 756

## Description

La présente invention concerne un système de production de biogaz comportant au moins une unité centrale de stockage de digestat liquide et de digestion complémentaire, au moins une unité centrale de stockage de biogaz, une pluralité de digesteurs anaérobies, un réseau d'alimentation de digestat liquide, un réseau d'évacuation de digestat liquide et un réseau d'évacuation de biogaz.

L'invention concerne également un procédé de production de biogaz.

Au sens de la présente invention, on entend par biomasse solide, des matières organiques d'origine végétales, animales, bactériologiques ou fongiques, contenant un taux de matière sèche supérieur à 12%, de préférence supérieur à 15%, qui permettent la production de biogaz.

La biomasse solide peut être fibreuse, notamment pailleuse, et/ou hétérogène, et/ou contenir des éléments indésirables, notamment des cailloux, du gravier, du sable, des barbelés, des ficelles, des pièces métalliques provenant d'outils agricoles ou encore des sacs plastiques, des éléments d'emballage et de conditionnement, ou encore des déchets urbains, éventuellement mal triés.

La méthanisation (encore appelée digestion anaérobie) est un procédé biologique naturel qui met en œuvre la dégradation des matières organiques par plusieurs types de micro-organismes, en particulier des bactéries, dans des conditions contrôlées et ceci en l'absence d'oxygène.

Un tel procédé conduit, d'une part, à la production de biogaz, qui correspond à un mélange gazeux composé majoritairement de méthane et de dioxyde de carbone, et, d'autre part, à la production d'un produit humide riche en matières organiques partiellement stabilisées appelé digestat, qui peut être ensuite utilisé comme amendement organique.

Le biogaz présente l'avantage d'être un gaz convertible en énergie renouvelable et peut par exemple servir dans la production d'électricité et/ou de chaleur ou dans la production de carburant. Le biogaz peut également être injecté dans un réseau de gaz naturel après épuration, c'est-à-dire après que le méthane présent dans le biogaz a été séparé du CO₂ et des autres gaz présents dans le biogaz

Le digestat peut subir un traitement de séparation de phases liquide/solide dont la fraction solide (appelé digestat solide) est plus riche en matières organiques et en éléments phosphatés et la fraction liquide (appelé digestat liquide) est plus riche en azote ammoniacal et en potassium. Les digestats liquides et solides peuvent être stockés, et/ou traités et/ou épandus séparément.

Ainsi le procédé biologique de méthanisation permet de valoriser des matières organiques, en produisant une énergie renouvelable, et de diminuer les émissions de gaz à effet de serre, en captant le méthane issu de la dégradation de ces matières, et en substituant l'emploi d'énergies fossiles et/ou d'engrais chimiques.

Les matières organiques, susceptibles de produire du biogaz par le biais d'une méthanisation, peuvent être des déchets provenant par exemple du secteur agricole et/ou agro-industriel et/ou être d'origine urbaine. En particulier, le fumier, provenant de l'exploitation agricole d'élevages, est une matière organique, issue des déjections animales mélangées à la paille des litières, qui constitue une source majeure et particulièrement intéressante dans la production de biogaz. A cet égard, la méthanisation dans le secteur agricole à partir de matières organiques, en particulier de pailles et/ou de fumier, représente une activité qui se développe de plus en plus à l'heure actuelle.

La méthanisation est couramment mise en œuvre à l'intérieur d'un réacteur appelé digesteur correspondant le plus souvent à une cuve, ayant une forme le plus souvent essentiellement cylindrique, mais parfois parallélépipédique, verticale ou horizontale, qui est destinée à contenir les matières organiques à traiter, en particulier du fumier et/ou des pailles et/ou des déchets d'origine industrielle et/ou d'origine urbaine, afin de produire du biogaz et du digestat.

Les différents procédés de méthanisation peuvent être répartis en deux grandes familles en fonction de la nature des déchets à dégrader, à savoir les procédés de méthanisation en voie liquide et les procédés de méthanisation en voie sèche.

En premier lieu, les procédés de méthanisation en voie liquide sont généralement mis en œuvre pour traiter des déchets dont le mélange a un taux moyen de matière sèche inférieur à 15%, correspondant par exemple à des mélanges de boues liquides ou de graisses ou des mélanges principalement composés de lisier. De tels procédés peuvent être aussi utilisés pour traiter des matières organiques solides qui ont été mélangées avec de l'eau ou du digestat liquide.

Les procédés de méthanisation en voie liquide consistent notamment à acheminer de manière généralement continue les matières organiques à traiter, le plus souvent au moyen de systèmes de pompage ou de vis sans fin à l'intérieur d'un digesteur qui est généralement hermétiquement fermé par l'intermédiaire d'un toit ou d'une membrane souple. Les matières organiques à traiter sont continuellement brassées à l'intérieur du digesteur, souvent par un ou plusieurs agitateurs, pouvant être à hélices ou à pâles, ou parfois par des injections de gaz, notamment de biogaz, afin d'éviter les phénomènes de décantation de la biomasse au fond du digesteur et/ou les phénomènes de flottation et de croûtage de la biomasse en surface du liquide dans le digesteur, et de favoriser la formation de biogaz par le contact entre la biomasse et les micro-organismes. Le digesteur peut éventuellement être chauffé dans des températures comprises entre 20 et 60°C, en particulier dans des températures comprises entre 35 et 55°C. Les matières organiques séjournent en moyenne plusieurs semaines dans le digesteur, typiquement pendant une période pouvant aller de 20 à 80 jours.

Le biogaz, produit au cours de cette réaction de méthanisation, est généralement stocké à pression atmosphérique dans une membrane souple étanche fixée au-dessus du digesteur. La membrane se présente alors sous la forme d'un dôme contenant un ciel gazeux au-dessus de la paroi verticale du digesteur. Plus rarement, le biogaz peut être stocké dans un gazomètre, qui est souvent une poche de stockage souple située à côté du digesteur.

Les procédés de méthanisation en voie liquide présentent notamment l'inconvénient que les biomasses à dégrader peuvent être difficiles à manipuler et à préparer pour leur acheminement vers le digesteur et nécessitent une dépense d'énergie importante pour être brassés de manière continue à l'intérieur du digesteur.

Dans les procédés de méthanisation en voie liquide qui incluent l'introduction de matières organiques solides, les matières organiques solides sont souvent broyées avant d'être incorporées dans la cuve de digestion afin de faciliter l'acheminement des matières organiques au sein du digesteur ainsi que leur brassage. Parfois, les matières organiques solides font l'objet d'un processus de tri préalable afin d'en retirer le plus possible d'objets indésirables.

Ces types de procédés de méthanisation en voie liquide continue présentent souvent des difficultés au niveau de l'alimentation de la biomasse car il est souvent nécessaire de traiter préalablement les biomasses avant leur introduction dans le digesteur, notamment en broyant la partie solide de la biomasse et en extrayant les matières indésirables. De plus, les systèmes de brassage au sein du digesteur peuvent être endommagés du fait de la nature des matières organiques ou des matières indésirables.

En second lieu, les procédés de méthanisation, dit par voie sèche, sont mis en œuvre pour traiter des déchets solides dont le mélange a un taux moyen de matière sèche généralement supérieur à 15%, notamment allant de 15% à 40%, correspondant par exemple à du fumier, de la paille, des déchets industriels ou urbains, ou des biomasses hétérogènes.

Un premier type de procédé de méthanisation par voie sèche consiste à acheminer en continu, notamment au moyen d'une trémie et d'un système de vis sans fin, ou plus rarement d'un système de pompage puissant, les matières organiques solides à traiter à l'intérieur d'un digesteur, le plus souvent disposé de manière horizontale. Les matières organiques solides à traiter sont brassées, le plus souvent lentement, à l'intérieur du digesteur qui peut être également chauffé dans des températures comprises entre 20 et 60°C, en particulier dans des températures comprises entre 35 et 55°C.

De plus, les matières organiques solides sont souvent broyées avant d'être incorporées dans la cuve de digestion afin de faciliter l'acheminement des matières organiques au sein du digesteur ainsi que leur brassage. Parfois les matières organiques solides font aussi l'objet d'un processus de tri préalable afin d'en retirer le plus possible d'objets indésirables.

Ce type de procédé de méthanisation en voie sèche continue présente souvent des difficultés au niveau de l'alimentation de la biomasse car il est souvent nécessaire de traiter préalablement les déchets avant leur introduction dans le digesteur, notamment en les broyant et en extrayant les matières indésirables. De plus, le système de brassage au sein du digesteur peut être endommagé du fait de la nature des matières organiques ou des matières indésirables.

Un second type de procédé de méthanisation en voie sèche est un procédé discontinu, ou en bâchées. Il consiste à acheminer, souvent au moyen d'engins mécaniques de chargement/déchargement, par exemple de type chargeur à godets, les matières organiques solides à l'intérieur de cellules de méthanisation qui sont fermées une fois remplies. Ces cellules sont généralement des sortes de garages équipés de portes que l'on referme une fois le chargement réalisé. Ce type de procédé est souvent dénommé procédé en garage. Les matières organiques sont ensuite aspergées ou douchées avec du digestat liquide, chargé en bactéries, afin de réaliser une percolation conduisant à la production de biogaz. Une fois la réaction terminée, les portes de chaque cellule sont ouvertes afin de récupérer le digestat solide, généralement au moyen des mêmes engins mécaniques que pour le chargement. Un tel procédé met généralement en œuvre plusieurs cellules de méthanisation ou digesteurs, alimentées en bâchées l'une après l'autre. Dans ce type de procédé, les cellules sont généralement destinées à fonctionner majoritairement en même temps même si elles ont été alimentées à des moments différents. Le biogaz, produit au cours de la réaction de méthanisation, est le plus souvent stocké séparément dans un gazomètre, souvent une poche de stockage souple située à côté des digesteurs.

Ce type de procédé en garage pose notamment le problème de la gestion du biogaz. Au début de la réaction de méthanisation, le biogaz produit dans chaque cellule est souvent mis en mélange avec l'air présent dans le garage avant fermeture de la porte du garage et après le chargement de la biomasse à traiter, ce qui a pour conséquence que, pendant le temps nécessaire au remplacement de l'air par du biogaz, un mélange air-biogaz est produit par la cellule. De même, en fin de réaction, le biogaz présent dans le garage doit être remplacé par de l'air avant ouverture de la porte du garage, ce qui a pour conséquence que, pendant le temps nécessaire au remplacement du biogaz par de l'air, un mélange air-biogaz est également produit par la cellule. Ces mélanges d'air et de biogaz produits en début et en fin de réaction de méthanisation ont pour conséquence que le biogaz produit par les procédés de méthanisation en garage est souvent mélangé avec de l'air.

Cette présence d'air dans le biogaz produit par les procédés en garage rend très difficile la valorisation du biogaz par injection du bio-méthane dans un réseau de gaz naturel. En effet, pour pouvoir être injecté dans un réseau de gaz naturel, le biogaz doit être épuré, c'est-à-dire que le méthane présent dans le biogaz doit être séparé du CO₂ et des autres gaz présents dans le biogaz. Or lorsque le biogaz contient de l'air, il contient en particulier de l'azote gazeux (N₂) qui peut s'avérer techniquement difficile à séparer du méthane.

De plus, dans un garage, la réaction de méthanisation n'est pas convenablement optimisée car la biomasse solide, introduite dans les cellules, n'est pas mélangée intimement avec le digestat liquide et souvent des chemins préférentiels de circulation du digestat liquide dans la biomasse solide se forment, empêchant une bonne diffusion du digestat liquide au sein la biomasse solide à traiter, et donc empêchant un contact intime entre les bactéries et la biomasse, réduisant sensiblement le taux de dégradation de la biomasse solide.

Plus généralement, un autre inconvénient majeur liés aux procédés de méthanisation en voie liquide et en voie sèche est l'émission de mauvaises odeurs pouvant survenir sur le lieu de production de biogaz notamment lors du stockage et du traitement préalable des biomasses solides, ainsi que lors des opérations de chargement et de déchargement de biomasse dans les unités de méthanisation.

Par exemple, les fumiers, les lisiers, et généralement tous les déchets issus des déjections animales, mais aussi les déchets organiques issus des activités industrielles, comme par exemple les déchets d'abattoirs, ainsi que les déchets organiques issus des municipalités ou de la grande distribution sont le plus souvent des déchets susceptibles d'émettre des odeurs fortes et désagréables au cours de leur stockage et des prétraitements dont ils font l'objet avant leur introduction dans l'unité de méthanisation.

Dans une moindre mesure, les opérations de traitement du digestat, c'est-à-dire après digestion anaérobie des matières organiques, peuvent également conduire à l'émission d'odeurs désagréables, en particulier au cours des opérations de séparation de phase, notamment en raison de l'évaporation de l'ammoniac contenu dans le digestat.

De fait, les émissions d'odeurs sont un enjeu majeur dans l'acceptation par le voisinage des projets de méthanisation, qu'ils soient réalisés par l'intermédiaire d'un procédé en voie sèche ou d'un procédé en voie liquide. Il s'agit le plus souvent de l'enjeu principal. En effet, les moyens mis en œuvre pour minimiser et contrôler les odeurs générées au cours des différentes étapes de méthanisation sur un territoire sont très onéreux en investissement et en exploitation : bâtiments de confinement des installations de prétraitements, systèmes d'aspiration de l'air souillé dans de grands volumes, systèmes d'épuration d'air par voie physico-chimiques, systèmes d'épuration d'air par voie biologiques, audits réalisés par des organismes agréés, réunions périodiques avec des associations de riverains afin d'évaluer et contrôler le niveau des mauvaises odeurs, etc.

Ainsi les matières organiques à traiter soulèvent souvent des difficultés au cours des différents procédés de méthanisation car ces biomasses présentent généralement l'inconvénient de comporter de nombreux éléments indésirables et/ou d'être difficiles à manipuler et/ou à broyer, ce qui rend difficiles les opérations de préparation de la biomasse (tri, broyage, pompage, transferts, etc.).

En effet, la paille, ainsi que le fumier, ou encore les biomasses hétérogènes, peuvent comporter des cailloux, du gravier, du sable, des barbelés, des ficelles, des pièces métalliques provenant d'outils agricoles ou encore des sacs plastiques, des éléments d'emballage ou de conditionnement. De tels éléments sont souvent difficiles à séparer de la biomasse à traiter et peuvent donc endommager les installations composant l'unité de méthanisation. A titre d'exemple, ces éléments indésirables peuvent endommager les pompes, les vis sans fin, les agitateurs et les broyeurs, conduisant ainsi au remplacement partiel ou total des pièces d'usure et/ou des pièces principales selon des fréquences élevées, engendrant des coûts de maintenance élevés.

De plus, la paille et les produits pailleux, tels que les fumiers, la menu-paille, les cannes de maïs, etc., sont délicats à manipuler car les produits pailleux sont abrasifs, ce qui peut abîmer, dégrader ou user les équipements employés au cours des différentes étapes des procédés de méthanisation (étapes de broyage, de triage, de pompage, de pressage et/ou de brassage).

De tels dégâts sont donc susceptibles d'entraîner une diminution de la production de biogaz, voire un arrêt de celle-ci, et de générer des manques à gagner et/ou une augmentation des coûts de maintenance et d'exploitation de l'installation.

En outre, une fois introduits, la paille et autres produits pailleux sont souvent difficiles à brasser dans le digesteur, car ils ont tendance à flotter et/ou à décanter et/ou à s'emmêler autour des agitateurs, ce qui engendre des dépenses d'énergie importantes, notamment des consommations électriques élevées. De telles dépenses énergétiques impactent négativement sur la rentabilité des installations.

Ainsi le fumier, la paille et les produits pailleux sont des matières organiques qui peuvent s'avérer difficiles à valoriser en vue d'une méthanisation.

Afin de remédier à certains de ces inconvénients, la demande de brevet FR 2 990 951 décrit notamment un procédé de préparation d'un substrat de méthanisation à partir de biomasse fibreuse solide, telle que le fumier et la paille, comprenant une étape d'immersion de la biomasse dans un liquide, en particulier de l'eau ou du digestat liquide, une étape d'hydrolyse et une étape de séparation de la matière sèche en suspension du liquide par pressage de manière à préparer un substrat de méthanisation. En particulier, ce procédé comprend une étape de broyage de la biomasse avant ou après immersion, par exemple mise en œuvre à l'aide d'une pompe broyeuse. Ce procédé a pour de but de retirer les éléments indésirables de la biomasse fibreuse solide par décantation et, par conséquent, d'optimiser la préparation du substrat de méthanisation avant de réaliser son acheminement dans le digesteur. Un système de production de biogaz avec une pluralité de digesteurs amovibles est décrit dans le document US 5958756 A.

Cependant, la biomasse fibreuse solide reste encore trop souvent difficile à manipuler au cours de ce procédé. En effet, la paille s'avère compliquée à compresser après immersion, ce qui peut engendrer des problèmes de serrage, de bouchage et de blocage au niveau des pompes et des presses et/ou des moyens de séparation.

Un tel procédé présente également l'inconvénient d'engendrer des dépenses d'énergie importantes afin de maintenir correctement la paille en suspension dans le liquide au cours de l'étape de dilution. En effet, on observe que la paille a tendance à s'emmêler, à s'agglomérer, à flotter et à ne pas se mélanger aisément dans le liquide.

De plus, ce procédé ne résout pas convenablement le problème survenant lors du brassage de la biomasse dans le digesteur. En effet, la paille reste encore trop difficile à brasser dans le digesteur car cette dernière flotte en surface et s'emmêle, ce qui conduit à un phénomène d'accumulation et de croûtage qui empêche notamment la bonne circulation du biogaz au sein de la cuve, ce qui complique sa récupération, et qui peut aboutir à la prise en masse complète de la biomasse solide présente dans le digesteur et la paralysie de celui-ci.

Ainsi les différentes unités de méthanisation mises en œuvre à l'heure actuelle présentent un certain nombre d'inconvénients quand elles doivent traiter en totalité ou en partie des biomasses solides, et en particulier des biomasses solides hétérogènes.

Au vu de ce qui précède, l'invention a notamment pour but de valoriser plus efficacement la biomasse solide par méthanisation tout en minimisant les dépenses d'énergie, en réduisant les dépenses de personnel d'exploitation, en rendant non nécessaire l'achat et l'installation de certains équipements de préparation et d'agitation de la biomasse solide, en diminuant les risques d'endommagement des installations composant l'unité de méthanisation et en diminuant les coûts de maintenance et de préparation de la biomasse solide.

La présente invention a donc notamment pour objet un système de production de biogaz comprenant :
- au moins une unité centrale de stockage de digestat liquide et de digestion complémentaire, constituée d'un ou plusieurs ouvrages, apte à contenir principalement du digestat liquide,
- au moins une unité centrale de stockage de biogaz, constitué d'un ou plusieurs ouvrages, apte à contenir principalement du biogaz,
- une pluralité de digesteurs anaérobies dont certains sont des digesteurs de biomasse solide, chacun desdits digesteurs de biomasse solide comprenant :
   - une cuve apte à contenir de la biomasse solide, comportant au moins une zone d'admission de la biomasse solide, située sur la partie supérieure de la cuve, sur laquelle un toit est apte à s'ouvrir et à se refermer, et comportant en outre au moins une zone d'évacuation de la biomasse solide digérée résiduelle, certaines desdites zones d'admission et d'évacuation de biomasse solide pouvant être communes, et
   - au moins une ouverture d'alimentation en digestat liquide, située sur la partie inférieure du digesteur anaérobie, reliée à au moins un circuit d'alimentation de digestat liquide, apte à permettre l'introduction de digestat liquide dans ledit digesteur de biomasse solide, et
   - au moins une ouverture d'évacuation de digestat liquide, située sur la partie supérieure du digesteur anaérobie, reliée à au moins un circuit d'évacuation de digestat liquide, apte à permettre l'évacuation de digestat liquide dudit digesteur de biomasse solide,
   - au moins une ouverture d'évacuation de biogaz, située sur la partie supérieure du digesteur anaérobie, reliée à au moins un circuit d'évacuation de biogaz, apte à permettre l'évacuation de biogaz depuis ledit digesteur de biomasse solide, et
   - au moins une ouverture de vidange de digestat liquide, située sur la partie inférieure du digesteur anaérobie, apte à permettre la vidange de digestat liquide dudit digesteur de biomasse solide, et
   - au moins une ouverture d'introduction et/ou d'évacuation d'air, située sur la partie supérieure du digesteur anaérobie, apte à permettre l'introduction et/ou l'évacuation d'air dans ledit digesteur de biomasse solide,
      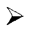 certaines ouvertures parmi lesdites ouvertures pouvant être communes, à l'exception des ouvertures d'alimentation et d'évacuation de digestat liquide qui sont distinctes, et
      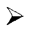 au moins un réseau d'alimentation de digestat liquide, incluant une pluralité de circuits d'alimentation de digestat liquide, reliant certaines ouvertures d'alimentation de digestat liquide de certains digesteurs de biomasse solide à l'unité centrale de stockage de digestat liquide et de digestion complémentaire, apte à permettre l'alimentation de digestat liquide, par voie directe ou indirecte, dans lesdits digesteurs depuis l'unité centrale de stockage de digestat liquide et de digestion complémentaire, et
      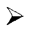 au moins un réseau d'évacuation de digestat liquide, incluant une pluralité de circuits d'évacuation de digestat liquide, reliant certaines ouvertures d'évacuation de digestat liquide de certains digesteurs de biomasse solide à l'unité centrale de stockage de digestat liquide et de digestion complémentaire, apte à permettre l'évacuation de digestat liquide, par voie directe ou indirecte, depuis lesdits digesteurs vers l'unité centrale de stockage de digestat liquide et de digestion complémentaire, et
      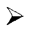 au moins un réseau d'évacuation de biogaz, incluant une pluralité de circuits d'évacuation de biogaz, reliant certaines ouvertures d'évacuation de biogaz de certains digesteurs de biomasse solide à l'unité centrale de stockage de biogaz, apte à permettre l'évacuation de biogaz, par voie directe ou indirecte, depuis lesdits digesteurs vers l'unité centrale de stockage de biogaz,
      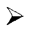 certaines parties desdits réseaux pouvant être communes, et
         - au moins un moyen de circulation de digestat liquide, de préférence une pompe, située sur le réseau d'alimentation de digestat liquide et/ou sur le réseau d'évacuation de digestat liquide, apte à permettre la circulation du digestat liquide à travers certains digesteurs de biomasse solide, et
         - au moins un dispositif de chauffage de digestat liquide, et
         - dont, au moins un digesteur de biomasse solide et au moins une unité centrale de stockage de digestat liquide et de digestion complémentaire sont calorifugée

Le système de production de biogaz selon l'invention présente l'avantage de valoriser plus efficacement la biomasse solide tout en réduisant les investissements en divers équipements, les dépenses énergétiques, de personnel et de maintenance des équipements, l'usure des matériels et les risques d'endommagement.

Le système de production de biogaz selon l'invention comprend une pluralité de digesteurs anaérobie de biomasse solide.

Par digesteur anaérobie de biomasse solide, on entend un digesteur apte à contenir de la biomasse solide et dans lequel une réaction de méthanisation est apte à se produire dans des conditions anaérobies.

De préférence, le ou les digesteurs anaérobies de biomasse solide sont conçus pour contenir de la biomasse solide.

En particulier, le ou les digesteurs anaérobies de biomasse solide sont immobiles, c'est-à-dire inamovibles.

Conformément à la présente invention, chaque digesteur anaérobie de biomasse solide du système de production de biogaz comprend une cuve apte à contenir de la biomasse solide, laquelle cuve comporte une ou plusieurs zone(s) d'admission de la biomasse solide. Ladite ou lesdites zone(s) d'admission, est (sont) située(s) en particulier sur au moins une partie de la surface supérieure de la cuve sur laquelle le toit s'ouvre et se referme, permettant l'incorporation de la biomasse solide à l'intérieur de la cuve.

La zone d'admission permet donc d'introduire directement la biomasse solide brute (i.e. non traitée) dans le digesteur sans avoir à effectuer une opération de séparation des éléments indésirables ou une dilution préalable dans un liquide ou un broyage, ce qui permet de diminuer les investissements dans les équipements de préparation de la biomasse solide, de diminuer les dépenses énergétiques, de maintenance et de personnel et les risques d'endommagement par rapport à des unités de méthanisation classiques.

En particulier, les digesteurs anaérobies du système selon l'invention permettent de s'affranchir d'un dispositif visant à séparer les éléments indésirables de la biomasse solide, d'un dispositif de broyage et d'un dispositif d'agitation de la biomasse dans le digesteur.

Après introduction de la biomasse solide, et fermeture du toit, la cuve des digesteurs est remplie avec du digestat liquide en provenance, directe ou indirecte, d'au moins une unité centrale de stockage de digestat liquide et de digestion complémentaire apte à stocker du digestat liquide. Après remplissage de la cuve, d'abord par de la biomasse solide, puis par du digestat liquide, la méthanisation se déroule par percolation du digestat liquide à travers la biomasse solide et diffusion du digestat liquide dans la biomasse solide, laquelle est totalement immergée (et éventuellement partiellement mise en suspension), dans des conditions anaérobies au sein de la cuve des digesteurs. Le biogaz se forme alors au cours de la méthanisation.

La digestion de la biomasse solide par percolation du digestat liquide à travers la biomasse solide et diffusion du digestat liquide dans la biomasse solide permet de s'affranchir de la nécessité d'agiter le mélange de biomasse solide et de digestat liquide dans la cuve du digesteur, évitant ainsi des investissements en moyens mécaniques d'agitation et des coûts d'exploitation.

L'ouverture d'évacuation du biogaz, située sur la partie supérieure du digesteur anaérobie, permet alors d'évacuer le biogaz, formé lors de l'étape de digestion de la biomasse, qui va ensuite être acheminé vers l'unité centrale de stockage de biogaz.

L'ouverture d'évacuation du digestat liquide permet quant à elle d'évacuer le digestat liquide qui va être acheminé vers l'unité centrale de stockage de digestat liquide et de digestion complémentaire.

Une fois la méthanisation terminée, une vidange aérobie du digestat liquide contenu dans la cuve du digesteur est réalisée, au cours de laquelle l'espace vacant laissé par cette vidange est rempli par de l'air, et l'accès à travers la zone d'admission permet alors de récupérer facilement la biomasse solide digérée résiduelle, une fois la digestion terminée, en minimisant l'énergie dépensée au cours de cette opération. Il s'agit donc d'un procédé de méthanisation discontinu, ou en bâchées.

En d'autres termes, la zone d'admission constitue un orifice d'entrée et de sortie de la biomasse solide situé au niveau de la surface supérieure de la cuve du digesteur selon l'invention.

Autrement dit, la ou les zones d'admission de biomasse solide est ou sont communes à la ou les zones d'évacuation de biomasse solide digérée résiduelle.

Ainsi certains digesteurs anaérobies de biomasse solide, de préférence les digesteurs anaérobies de biomasse solide, sont reliés, par le biais d'un réseau d'alimentation en digestat liquide et d'un réseau d'évacuation de digestat liquide à l'unité centrale de stockage de digestat liquide et de digestion complémentaire, et, par le biais d'un réseau d'évacuation de biogaz, à l'unité centrale de stockage de biogaz.

### Réseau d'alimentation en digestat liquide

Au sens de la présente invention, un réseau d'alimentation en digestat liquide comprend une pluralité de circuits d'alimentation de digestat liquide aptes à relier au moins une ouverture d'alimentation en digestat liquide d'un ou plusieurs digesteurs anaérobies de biomasse solide à l'unité centrale de stockage de digestat liquide et de digestion complémentaire ce qui permet d'alimenter, par voie directe ou indirecte, en digestat liquide le ou les digesteurs anaérobies de biomasse solide.

Par réseau d'alimentation de digestat liquide apte à permettre l'alimentation de digestat liquide par voie directe dans certains digesteurs de biomasse solide depuis une unité centrale de stockage de digestat liquide et de digestion complémentaire, on entend au sens de l'invention que lesdits digesteurs sont alimentés directement par du digestat liquide en provenance de ladite unité centrale de stockage de digestat liquide et de digestion complémentaire. Il s'agit d'une liaison en parallèle.

En particulier, pour chaque digesteur relié en parallèle à ladite unité centrale de stockage de digestat liquide et de digestion complémentaire, l'alimentation en digestat liquide se fait directement depuis ladite unité centrale de stockage de digestat liquide et de digestion complémentaire, sans transiter par un autre digesteur anaérobie.

Par réseau d'alimentation de digestat liquide apte à permettre l'alimentation de digestat liquide par voie indirecte dans certains digesteurs depuis une unité centrale de stockage de digestat liquide et de digestion complémentaire, on entend que certains digesteurs de biomasse solide sont alimentés par du digestat liquide en provenance de ladite unité centrale de stockage de digestat liquide et de digestion complémentaire à travers un ou plusieurs autres digesteurs précédents. Il s'agit d'une liaison en série.

En particulier, pour chaque digesteur de biomasse solide relié en série à ladite unité centrale de stockage de digestat liquide et de digestion complémentaire, l'alimentation en digestat liquide se fait de façon indirecte depuis ladite unité centrale de stockage de digestat liquide et de digestion complémentaire, et le digestat liquide transite par un ou plusieurs autres digesteurs anaérobies situés directement ou non aval avant d'alimenter ledit digesteur anaérobie.

Conformément à ce mode de réalisation, l'alimentation, par voie indirecte, en digestat liquide d'un digesteur anaérobie provient du digesteur anaérobie précédent, lequel digesteur peut être à son tour soit relié directement à ladite unité centrale de digestion complémentaire et de stockage du digestat liquide, soit relié à un autre digesteur anaérobie, et ainsi de suite.

De préférence, le ou les réseau(x) d'alimentation de digestat liquide comprennent des liaisons en parallèle, c'est-à-dire que le réseau d'alimentation de digestat liquide est apte à permettre l'alimentation de digestat liquide par voie directe.

De préférence, le réseau d'alimentation en digestat liquide relie l'ensemble des digesteurs anaérobies du système de production de biogaz à l'unité centrale de stockage de digestat liquide et de digestion complémentaire.

### Réseau d'évacuation en digestat liquide

Au sens de la présente invention, un réseau d'évacuation en digestat liquide comprend une pluralité de circuits d'évacuation de digestat liquide aptes à relier au moins une ouverture d'évacuation en digestat liquide d'un ou plusieurs digesteurs anaérobies de biomasse solide à l'unité centrale de stockage de digestat liquide et de digestion complémentaire ce qui permet d'évacuer, par voie directe ou indirecte, le digestat liquide depuis le ou les digesteurs anaérobies de biomasse solide vers ladite unité centrale.

Par réseau d'évacuation de digestat liquide, apte à permettre l'évacuation de digestat liquide par voie directe de certains digesteurs de biomasse solide vers une unité centrale de stockage de digestat liquide et de digestion complémentaire, on entend au sens de l'invention que du digestat liquide contenu dans lesdits digesteurs est évacué directement vers ladite unité centrale de stockage de digestat liquide et de digestion complémentaire. Il s'agit d'une liaison en parallèle.

En particulier, depuis chaque digesteur relié en parallèle à ladite unité centrale de stockage de digestat liquide et de digestion complémentaire, l'évacuation de digestat liquide se fait directement vers ladite unité centrale de stockage de digestat liquide et de digestion complémentaire, sans transiter par un autre digesteur anaérobie.

Par réseau d'évacuation de digestat liquide, apte à permettre l'évacuation de digestat liquide par voie indirecte de certains digesteurs de biomasse solide vers une unité centrale de stockage de digestat liquide et de digestion complémentaire, on entend au sens de l'invention que du digestat liquide contenu dans lesdits digesteurs est évacué vers ladite unité centrale de stockage de digestat liquide et de digestion complémentaire à travers un ou plusieurs autres digesteurs. Il s'agit d'une liaison en série.

En particulier, pour chaque digesteur de biomasse solide relié en série à ladite unité centrale de stockage de digestat liquide et de digestion complémentaire, l'évacuation de digestat liquide se fait de façon indirecte vers ladite unité centrale de stockage de digestat liquide et de digestion complémentaire, et le digestat liquide transite par un ou plusieurs autres digesteurs anaérobies avant d'alimenter ladite unité centrale de stockage de digestat liquide et de digestion complémentaire.

Conformément à ce mode de réalisation, l'évacuation, par voie indirecte, de digestat liquide d'un digesteur anaérobie se fait vers le digesteur anaérobie suivant, lequel digesteur peut être à son tour soit être relié directement à l'unité centrale de digestion complémentaire et de stockage du digestat liquide, soit être relié à un autre digesteur anaérobie, et ainsi de suite.

De préférence, le ou les réseau(x) d'évacuation de digestat liquide comprennent des liaisons en parallèle c'es-à-dire que le réseau d'évacuation de digestat liquide est apte à permettre l'évacuation de digestat liquide par voie directe.

De préférence, le réseau d'évacuation de digestat liquide relie l'ensemble des digesteurs anaérobies du système de production de biogaz à l'unité centrale de stockage de digestat liquide et de digestion complémentaire.

### Réseau d'évacuation en biogaz

Au sens de la présente invention, un réseau d'évacuation de biogaz comprend une pluralité de circuits d'évacuation de biogaz aptes à relier au moins une ouverture d'évacuation en biogaz d'un ou plusieurs digesteurs anaérobies de biomasse solide à l'unité centrale de stockage de biogaz ce qui permet d'évacuer, par voie directe ou indirecte, le biogaz depuis le ou les digesteurs anaérobies de biomasse solide vers ladite unité centrale.

Par réseau d'évacuation de biogaz apte à permettre l'évacuation de biogaz par voie directe de certains digesteurs de biomasse solide vers une unité centrale de stockage de biogaz, on entend au sens de l'invention que du biogaz contenu dans lesdits digesteurs est évacué directement vers ladite unité centrale de stockage de biogaz et de digestion complémentaire. Il s'agit d'une liaison en parallèle.

En particulier, depuis chaque digesteur relié en parallèle à ladite unité centrale de stockage de biogaz, l'évacuation de biogaz se fait directement vers ladite unité centrale de stockage de biogaz, sans transiter par un autre digesteur anaérobie.

Par réseau d'évacuation de biogaz apte à permettre l'évacuation de biogaz par voie indirecte de certains digesteurs de biomasse solide vers une unité centrale de stockage de biogaz, on entend au sens de l'invention que du biogaz contenu dans lesdits digesteurs est évacué vers ladite unité centrale de stockage de biogaz à travers un ou plusieurs autres digesteurs. Il s'agit d'une liaison en série.

Conformément à ce mode de réalisation, pour chaque digesteur de biomasse solide relié en série à ladite unité centrale de stockage de biogaz, l'évacuation

En particulier, pour chaque digesteur de biomasse solide relié en série à ladite unité centrale de stockage de biogaz, l'évacuation de biogaz se fait de façon indirecte vers ladite unité centrale de stockage de biogaz, et le biogaz transite par un ou plusieurs autres digesteurs anaérobies avant d'alimenter ladite unité centrale de stockage de biogaz.

Conformément à ce mode de réalisation, l'évacuation, par voie indirecte, de biogaz d'un digesteur anaérobie se fait vers le digesteur anaérobie suivant, lequel digesteur peut être à son tour soit reliée directement à l'unité centrale de digestion complémentaire et de stockage du biogaz, soit reliée à un autre digesteur anaérobie, et ainsi de suite.

De préférence, le ou les réseau(x) d'évacuation de biogaz comprennent des liaisons en parallèle c'est-à-dire que le réseau d'évacuation de biogaz est apte à permettre l'évacuation de biogaz par voie directe.

De préférence, le réseau d'évacuation de biogaz relie l'ensemble des digesteurs anaérobies du système de production de biogaz à l'unité centrale de stockage de biogaz.

### Parties communes aux réseaux

Selon certains modes de réalisation, le réseau d'alimentation en digestat liquide, le réseau d'évacuation en digestat liquide et le réseau d'évacuation en biogaz présentent des parties communes, notamment un ou plusieurs circuits communs.

De préférence, le réseau d'évacuation en digestat liquide et le réseau d'évacuation en biogaz présentent des parties communes, notamment un ou plusieurs circuits en communs, c'est-à-dire un ou plusieurs circuits d'évacuation de digestat liquide et de biogaz.

### Moyen de circulation de digestat liquide

Le système de production de biogaz comprend au moins un moyen de circulation situé sur le réseau d'alimentation en digestat liquide et/ou sur le réseau d'évacuation de digestat liquide.

De préférence, le moyen de circulation de digestat liquide est une pompe, plus préférentiellement située sur le réseau d'alimentation de digestat liquide.

### Dispositif de chauffage de digestat liquide

Le système de production de biogaz comprend en outre au moins un dispositif de chauffage de digestat liquide.

De préférence, l'unité centrale de stockage du digestat liquide et de digestion complémentaire peut être équipée d'au moins un dispositif de chauffage du digestat liquide qu'elle contient.

De cette façon, le digestat liquide qui est destiné à alimenter le ou les digesteur(s) anaérobie(s), de préférence le ou les digesteur(s) anaérobies, est de préférence maintenu à une température comprise entre 30 et 55°C, et avantageusement entre 35 et 40°C, régime mésophile, ou à une température comprise entre 45 et 55°C, régime thermophile.

L'alimentation en digestat liquide chaud, continue ou séquentielle, du ou des digesteur(s) anaérobies par l'unité centrale de digestion complémentaire et de stockage du digestat liquide, permet le maintien dans le ou les digesteur(s) anaérobies d'une température de réaction élevée semblable à la température du digestat liquide dans l'unité centrale de digestion et de stockage du digestat liquide, ce qui favorisera la réaction de digestion anaérobie dans chacun des digesteurs anaérobies, de préférence en mode mésophile ou thermophile.

En outre, au moins un digesteur anaérobie de biomasse solide et au moins une unité centrale de stockage de digestat liquide et de digestion complémentaire sont calorifugés.

### Unité centrale de stockage de digestat liquide et de digestion complémentaire

L'unité centrale de stockage de digestat liquide et de digestion complémentaire est une unité apte à stocker du digestat liquide qui, conformément à la présente invention, alimente en digestat liquide le ou les digesteurs anaérobies, notamment les digesteurs anaérobies de biomasse solide.

L'unité centrale de stockage de digestat liquide et de digestion complémentaire récupère également le digestat liquide évacué depuis le ou les digesteurs anaérobies, notamment les digesteurs anaérobies de biomasse solide.

En outre, une digestion complémentaire peut avoir lieu au sein de l'unité centrale de stockage de digestat liquide et de digestion complémentaire.

L'unité centrale de stockage de digestat liquide et de digestion complémentaire a aussi de préférence une fonction supplémentaire de production de biogaz.

En effet, durant l'étape de digestion, la recirculation du digestat liquide provenant du ou des digesteurs anaérobies vers l'unité centrale de stockage du digestat liquide et digestion complémentaire, après diffusion du digestat liquide dans la biomasse solide, peut provoquer des entrainements avec le digestat liquide de matières fines solides biodégradables non encore digérées et/ou des entrainements de substances dissoutes biodégradables non encore digérées, comme par exemple des acides gras volatiles n'ayant pas eu le temps d'être transformés en biogaz dans le digesteur anaérobie.

Ainsi, la méthanisation résiduelle de ces matières se produit dans l'unité centrale de digestion complémentaire et de stockage du digestat liquide.

De préférence, l'unité centrale de stockage du digestat liquide et de digestion complémentaire ne contient pas de biomasse solide ou dans des quantités très faibles.

Selon une caractéristique de l'invention, certains desdits digesteurs de biomasse solide sont surmontés d'un moyen de préhension apte à permettre l'alimentation de la biomasse solide.

En d'autres termes, le moyen de préhension est apte à charger de la biomasse solide dans certains digesteurs de biomasse solide sur le lieu d'implantation du système de production de biogaz.

De préférence, le moyen de préhension est apte à charger de la biomasse solide dans au moins un digesteur anaérobie et à décharger la biomasse solide digérée résiduelle, une fois la digestion terminée, à travers la zone d'admission du digesteur.

Le moyen de préhension permet donc de saisir la biomasse solide et de l'incorporer dans le digesteur sans avoir à effectuer une étape de tri ou de séparation préalable des éléments indésirables susceptibles d'endommager les installations composant l'unité de méthanisation, et sans avoir à effectuer une étape de broyage ni de dilution de la biomasse solide.

Le moyen de préhension permet aussi de récupérer la biomasse solide digérée résiduelle une fois la digestion terminée, c'est-à-dire le digestat solide, et de l'évacuer du digesteur.

Ainsi le moyen de préhension permet d'alimenter et de décharger le digesteur par le haut à travers la zone d'admission (respectivement d'évacuation) située sur au moins une partie de la surface supérieure de la cuve sur laquelle se referme le toit.

Le moyen de préhension peut également servir à ouvrir le toit du digesteur avant d'alimenter le digesteur en biomasse solide ou de décharger la biomasse solide.

Le moyen de préhension peut également servir à fermer le toit du digesteur après les opérations d'alimentation du digesteur en biomasse solide ou de déchargement du digestat solide.

Le moyen de préhension est préférentiellement un grappin apte à saisir la biomasse solide par le biais d'une pluralité de crochets ou une benne apte à saisir de la biomasse plus pâteuse par le biais de godets.

Selon une caractéristique de l'invention, dans certains desdits digesteurs de biomasse solide, au moins l'une des ouvertures d'évacuation du digestat liquide comprend au moins un élément de séparation perforé d'évacuation du digestat liquide, situé en amont de ladite ouverture d'évacuation du digestat liquide, apte à séparer, d'une part, le digestat liquide évacué et, d'autre part, la biomasse solide.

En particulier, l'élément de séparation perforé d'évacuation du digestat liquide permet l'évacuation du digestat liquide au cours de l'étape de digestion de la biomasse solide tout en retenant la biomasse solide au sein du digesteur.

En outre, l'évacuation du biogaz peut se faire et se fait de préférence concomitamment à l'évacuation et la recirculation du digestat liquide vers l'unité centrale de digestion complémentaire et de stockage du digestat liquide.

Selon une caractéristique de l'invention, dans certains desdits digesteurs de biomasse solide, au moins l'une des ouvertures de vidange du digestat liquide comprend au moins un élément de séparation perforé de vidange du digestat liquide, situé en amont de ladite ouverture de vidange du digestat liquide, apte à séparer, d'une part, le digestat liquide vidangé et, d'autre part, la biomasse solide.

En particulier, l'élément de séparation perforé de vidange du digestat liquide permet la circulation du digestat liquide lors des étapes de vidanges anaérobies et aérobies du digesteur tout en retenant la biomasse solide dans la cuve desdits digesteurs anaérobies de biomasse solide.

De préférence, certains éléments de séparation perforés sont des plaques perforées.

Plus préférentiellement, l'élément de séparation perforé d'évacuation du digestat liquide et l'élément de séparation perforé de vidange du digestat liquide sont des plaques perforées.

En particulier, l'élément de séparation perforé de vidange du digestat liquide est un plancher perforé pouvant être situé sur la totalité ou une partie du sol du digesteur anaérobie.

Selon une caractéristique de l'invention, sur certains desdits digesteur de biomasse solide:
- la ou les ouverture(s) d'alimentation en digestat liquide, est (sont) munie(s) d'un moyen d'obstruction, de préférence une vanne, apte à obturer ou permettre le passage du digestat liquide entre l'extérieur et l'intérieur du digesteur, et/ou
- la ou les ouverture(s) d'évacuation de digestat liquide, est (sont) munie(s) d'un moyen d'obstruction, de préférence une vanne, apte à obturer ou permettre le passage du digestat liquide entre l'intérieur et l'extérieur du digesteur, et/ou
- la ou les ouverture(s) d'évacuation de biogaz, est (sont) munie(s) d'un moyen d'obstruction, de préférence une vanne, apte à obturer ou permettre le passage du biogaz entre l'intérieur et l'extérieur du digesteur, et/ou
- la ou les ouverture(s) de vidange de digestat liquide, est (sont) munie(s) d'un moyen d'obstruction, de préférence une vanne, apte à obturer ou permettre le passage du digestat liquide entre l'intérieur et l'extérieur du digesteur, et/ou
- la ou les ouverture(s) d'introduction et/ou d'évacuation d'air, est (sont) munie(s) d'un moyen d'obstruction, de préférence une vanne, apte à obturer ou permettre le passage de l'air entre l'intérieur et l'extérieur du digesteur.

En particulier, le ou les moyens d'obstruction, situé(s) sur la ou les ouvertures d'alimentation en digestat liquide, est ou sont apte(s) à permettre l'alimentation en digestat liquide du ou des digesteur(s) en provenance du réseau d'alimentation de digestat liquide.

En particulier, le ou les moyens d'obstruction, situé(s) sur la ou les ouvertures d'évacuation de digestat liquide, est ou sont apte(s) à permettre l'évacuation du digestat liquide depuis le (ou lesdits) digesteur(s) vers le réseau d'évacuation du digestat liquide.

En particulier, le ou les moyens d'obstruction, situé(s) sur la ou les ouvertures d'évacuation de biogaz, est ou sont apte(s) à permettre l'évacuation du biogaz depuis le (ou lesdits) digesteur(s) vers le réseau d'évacuation de biogaz.

En particulier, le ou les moyens d'obstruction, situé(s) sur la ou les ouverture(s) de vidange de digestat liquide, est ou sont apte(s) à permettre la vidange du digestat liquide depuis le (ou lesdits) digesteur(s) vers l'unité centrale de stockage de digestat liquide et de digestion complémentaire.

En particulier, le ou les moyens d'obstruction, situé(s) sur la ou les ouverture(s) d'introduction et/ou d'évacuation d'air, est ou sont apte(s) à permettre l'introduction ou l'évacuation de l'air dans le digesteur.

De préférence, lorsque certaines ouvertures sont communes alors les moyens d'obstruction desdites ouvertures sont également communs.

De préférence, lorsque l'ouverture d'évacuation de digestat liquide et l'ouverture d'évacuation de biogaz sont communs alors le ou les moyens d'obstruction, situé(s) sur chacune desdites ouvertures, sont communs.

Ainsi le ou les moyens d'obstruction, situé(s) sur la ou les ouvertures d'évacuation de digestat liquide et de biogaz, est ou sont apte(s) à permettre l'évacuation du digestat liquide et du biogaz vers le réseau d'évacuation de digestat liquide et le réseau d'évacuation de biogaz, notamment vers leurs parties communes.

De préférence, les moyens d'obstructions présents dans le système de production de biogaz sont des vannes.

Selon une caractéristique de l'invention, certains desdits digesteurs anaérobies de biomasse solide comportent un joint liquide positionné autour du toit de la cuve, apte à empêcher la sortie du biogaz contenu dans ledit digesteur lorsque le toit est fermé, et apte à empêcher les entrées d'air dans ledit digesteur lorsque le toit est fermé.

De préférence, le joint liquide est positionné autour du toit de la cuve des digesteurs anaérobies de biomasse solide.

Le joint liquide permet d'assurer une meilleure étanchéité du toit et d'éviter les fuites éventuelles de biogaz. De préférence, le joint liquide est une gouttière remplie d'eau.

En particulier, un robinet permet l'alimentation continue en eau du joint liquide afin de minimiser l'évaporation du joint.

Selon une caractéristique de l'invention, certains ouvrages composant l'unité centrale de stockage de digestat liquide et de digestion complémentaire, d'une part, et certains ouvrages composant l'unité centrale de stockage de biogaz, d'autre part, sont communs.

En variante, l'unité centrale de stockage de digestat liquide et de digestion complémentaire et l'unité centrale de stockage de biogaz constituent deux ouvrages distincts.

L'unité centrale de stockage du digestat liquide et de digestion complémentaire peut aussi correspondre à un digesteur apte à stocker conjointement du digestat liquide et du biogaz ou à deux cuves distinctes capables de stocker séparément du digestat liquide et du biogaz.

Dans certains cas, l'unité centrale de stockage du digestat liquide et de digestion complémentaire peut comporter plusieurs digesteurs aptes à stocker conjointement du digestat liquide et du biogaz ou plusieurs cuves distinctes capables de stocker séparément du digestat liquide et du biogaz.

Dans certains cas, l'unité centrale de stockage de digestat liquide et de digestion complémentaire peut notamment être constituée de plusieurs digesteurs anaérobies amovibles, aptes à stocker conjointement du digestat liquide et du biogaz. Dans ce cas, le biogaz peut avantageusement être stocké dans un ou plusieurs réservoirs souples dédiés et séparés.

De préférence, l'unité centrale de stockage de digestat liquide et de digestion complémentaire, d'une part, et l'unité centrale de stockage de biogaz, d'autre part, sont communes et constituent une seule et même unité centrale de stockage de digestat liquide et de biogaz et de digestion complémentaire.

Ainsi l'unité centrale de stockage du digestat liquide et de digestion complémentaire correspond à un digesteur apte à stocker conjointement du digestat liquide et du biogaz et constitué d'un seul ouvrage, en particulier comprenant du digestat liquide et du biogaz.

Selon une caractéristique de l'invention, sur certains des digesteurs de biomasse solide, au moins une ouverture d'évacuation de digestat liquide et une ouverture d'évacuation de biogaz sont communes et sont reliées à au moins un circuit d'évacuation d'un mélange de digestat liquide et de biogaz, lequel circuit est connecté à un moyen de séparation apte à séparer le biogaz et le digestat liquide.

Ainsi le digestat liquide et le biogaz sont évacués de manière concomitante à travers l'ouverture d'évacuation de digestat liquide et de biogaz pour être acheminés vers un élément apte à séparer le biogaz et le digestat liquide de manière à les transférer respectivement vers l'unité de stockage de digestat liquide et de digestion complémentaire et l'unité de stockage de biogaz.

L'invention concerne également un procédé de production de biogaz comprenant au moins les étapes successives suivantes :
- une étape d'alimentation en biomasse solide, mise en œuvre par au moins un moyen de préhension, à travers une zone d'admission située au niveau de la partie supérieure d'une cuve d'au moins un digesteur de biomasse solide calorifugé, tel que défini précédemment,
- une étape de remplissage de la cuve dudit digesteur, avec du digestat liquide pour immerger la biomasse solide,
- une étape de digestion, constituée d'une percolation du digestat liquide à travers la biomasse solide contenue dans ledit digesteur et d'une diffusion du digestat liquide dans ladite biomasse solide dans des conditions anaérobies pour générer puis récupérer du biogaz, et
- une étape de vidange aérobie du digestat liquide de la cuve du digesteur, et
- une étape d'évacuation de la biomasse solide digérée résiduelle, mise en œuvre par au moins un moyen de préhension, à travers une zone d'évacuation de la biomasse solide du digesteur.

En d'autres termes, la cuve d'au moins un digesteur anaérobie est alimentée par le haut en biomasse solide à travers une zone sur laquelle le toit du digesteur s'ouvre et se referme.

Le procédé de production du biogaz présente donc l'avantage de ne pas nécessiter une étape de préparation et/ou de séparation des éléments indésirables et/ou de broyage et/ou de dilution préalable de la biomasse solide ni de nécessiter de brassage de la biomasse solide dans le digesteur, ce qui permet de minimiser les investissements en équipements et les dépenses énergétiques associées à de telles étapes ainsi que les risques éventuels d'endommagement des équipements du digesteur ou des équipements mis en œuvre au cours du procédé et de diminuer les coûts de personnel et de maintenance liés aux différentes étapes évitées.

Selon une caractéristique, entre l'étape de digestion de la biomasse solide et l'étape de vidange aérobie du digestat liquide, le procédé comprend au moins les étapes suivantes :
- une étape de vidange anaérobie du digestat liquide de la cuve du digesteur, durant laquelle le digestat liquide, contenu dans le digesteur, est vidangé. De préférence, cette vidange consiste en un pompage à partir du bas de la cuve du digesteur vers l'unité de digestion principale à travers l'élément de séparation inférieur. Durant cette étape, l'espace vacant laissé par l'évacuation du digestat liquide est rempli par du biogaz en provenance de l'unité de digestion principale. A l'issue de cette étape la biomasse solide partiellement ou totalement digérée vient reposer sur le fond de la cuve du digesteur, et
- une étape de re-remplissage de la cuve du digesteur avec du digestat liquide. De préférence, le digestat liquide provient de l'unité de digestion principale après vidange anaérobie.

Selon une caractéristique, entre l'étape de re-remplissage de la cuve du digesteur avec un digestat liquide et l'étape de vidange aérobie de la biomasse liquide, le procédé comprend en outre au moins une étape de digestion constituée par une percolation du digestat liquide dans la biomasse solide et une diffusion du digestat liquide dans la biomasse solide pour générer puis récupérer le biogaz à travers la zone d'évacuation du biogaz située dans la partie supérieure du digesteur.

En particulier, le procédé comporte une étape d'ouverture du toit d'au moins un digesteur anaérobie avant l'étape d'alimentation en biomasse solide afin de permettre le remplissage par le haut de la cuve dudit digesteur anaérobie avec de la biomasse solide.

La zone d'admission est ainsi située sur au moins une partie de la surface supérieure de la cuve sur laquelle le toit se referme.

Selon un mode de réalisation, le procédé comporte une étape de fermeture du toit sur la zone d'admission du digesteur préalablement à l'étape de remplissage de la cuve avec du digestat liquide.

Optionnellement, le moyen de préhension peut également ouvrir et éventuellement fermer le toit du digesteur.

Le digestat liquide est ensuite introduit à l'intérieur de la cuve par le biais d'un ou plusieurs moyens de circulation, de préférence un ou plusieurs pompes d'alimentation, en provenance de l'unité de stockage du digestat liquide et de digestion complémentaire.

Selon un mode de réalisation, après l'étape de remplissage de la cuve par la biomasse solide à traiter et du digestat liquide, le moyen d'obstruction, de préférence une vanne, situé sur l'ouverture d'introduction et/ou d'évacuation d'air, est en position fermée de manière à ce que la réaction de méthanisation se déroule dans des conditions anaérobies.

L'étape de percolation conduit, au niveau de la partie supérieure du digesteur, à la formation d'un flux de biogaz et de digestat liquide.

La percolation est assurée par la mise en œuvre d'une ou plusieurs pompes de circulation située(s) entre l'unité de stockage de digestat liquide et de digestion complémentaire et la cuve du digesteur.

En particulier, la ou les pompe(s) de circulation permet(tent) d'assurer une pression du digestat liquide sur la biomasse solide l'obligeant à la traverser ce qui favorise un contact intime entre les bactéries contenues notamment dans le digestat liquide et la biomasse solide à dégrader.

La percolation s'accompagne d'une diffusion du digestat liquide dans la biomasse solide, laquelle est favorisée par la pression exercée par le pompage du digestat liquide sur la biomasse solide. Cette diffusion favorise le contact intime entre les bactéries et la biomasse solide à traiter.

De plus, le pompage et la circulation forcée du digestat liquide entre l'unité de stockage de digestat liquide et de digestion complémentaire et le digesteur présentent l'avantage de faire circuler une très grande quantité de digestat liquide à travers un volume plus faible de biomasse solide à traiter ce qui limite les risques d'acidose dans le digesteur, aux alentours de la biomasse solide à traiter.

En effet, dans les autres procédés de méthanisation, des quantités trop importantes de biomasse à traiter dans le digesteur peuvent conduire à une réaction d'acidogénèse excessive, pouvant provoquer une acidification du digestat liquide, ou acidose, laquelle entraine une inhibition partielle, voire totale, de la méthanogénèse et donc de la production de méthane. En synthèse, une telle réaction est provoquée lorsque la production d'acides gras volatils, induite par des bactéries acidogènes, est plus importante que la capacité de transformation de ces acides gras en méthane par les bactéries méthanogènes.

Selon un mode de réalisation, le procédé comprend, au moment de l'évacuation du biogaz vers l'unité centrale de stockage de biogaz et de la recirculation du digestat liquide vers l'unité centrale de stockage de digestat liquide et de digestion complémentaire, une étape de séparation, mise en œuvre au moyen d'au moins un élément de séparation perforé d'évacuation du digestat liquide, de préférence une plaque ou une grille perforée, permettant à la fois d'extraire le mélange composé de biogaz et de digestat liquide vers l'unité de digestion principale et de retenir dans le digesteur la biomasse fibreuse solide qui se trouve en suspension dans la cuve.

En particulier, pendant la phase de digestion anaérobie, le ou les moyens d'obstructions, situé(s) sur la ou les ouvertures d'évacuation de biogaz et la ou les ouvertures d'évacuation de digestat liquide, est ou sont en position ouverte afin de faire circuler le biogaz et le digestat vers l'unité de digestion principale. Le ou les moyens d'obstructions est (ou sont) notamment situés en aval de l'élément de séparation supérieur et en amont ou sur les circuits d'évacuation du biogaz et du digestat liquide.

Selon un mode de réalisation, l'étape de vidange anaérobie du digestat liquide est mise en œuvre en vidant par le bas la cuve du digestat liquide ce qui provoque le tassement de la biomasse solide au fond de la cuve et permet d'éliminer les chemins préférentiels de percolation ainsi que les poches de biogaz bloquées dans la biomasse.

L'étape de vidange anaérobie peut être mise en œuvre en aspirant le digestat liquide par le biais de la ou les pompe(s) située(s) entre la cuve du digesteur et l'unité de stockage de digestat liquide et de digestion complémentaire.

L'aspiration du digestat liquide peut de préférence avoir lieu en aval de l'élément perforé de vidange de digestat liquide pour éviter que la biomasse solide soit entrainée avec le digestat liquide vers l'unité de stockage de digestat liquide et de digestion complémentaire.

L'étape de vidange anaérobie permet au biogaz en provenance de l'unité de stockage de biogaz de remplir l'ensemble du volume libéré dans la cuve en remplacement du digestat liquide qui est aspiré par la pompe vers l'unité de stockage de digestat liquide et de digestion complémentaire.

Après l'étape de vidange anaérobie, une étape de re-remplissage de la cuve avec du digestat liquide est à nouveau mise en œuvre afin de poursuivre la réaction de méthanisation.

De la même façon que précédemment, l'étape de digestion conduit à la formation de biogaz, récupéré par le biais de l'élément de séparation perforé d'évacuation de digestat liquide au niveau de la partie supérieure du digesteur.

Selon un mode de réalisation, l'étape de vidange aérobie du digestat liquide de la cuve du digesteur est réalisée à la fin du cycle de méthanisation.

Une telle étape est mise en œuvre en fermant le ou les moyens d'obstruction, situé sur la ou les ouvertures d'évacuation de digestat liquide et la ou les ouvertures d'évacuation de biogaz, et en ouvrant le ou les moyens, situé(s) sur la ou les ouvertures d'introduction et/ou d'évacuation d'air dans le digesteur et en vidant la cuve du digestat liquide depuis le bas de la cuve du digesteur vers l'unité de stockage de digestat liquide et de digestion complémentaire.

L'étape de vidange aérobie est mise en œuvre en aspirant le digestat liquide par le biais de la ou les pompe(s) située(s) entre la cuve du digesteur et l'unité de stockage de digestat liquide et de digestion complémentaire.

L'aspiration du digestat liquide peut avoir lieu de préférence en aval de l'élément perforé de vidange de digestat liquide pour éviter que la biomasse solide soit entrainée avec le digestat liquide vers l'unité de digestion principale.

L'étape de vidange aérobie permet à l'air en provenance de l'extérieur de remplir l'ensemble du volume libéré dans la cuve en remplacement du digestat liquide qui est aspiré par la pompe vers l'unité de stockage de digestat liquide et de digestion complémentaire.

Selon un mode de réalisation, le procédé comporte une étape d'ouverture du toit sur la zone d'admission de la biomasse solide préalablement à l'étape d'évacuation du digestat solide, correspondant à la biomasse solide digérée résiduelle après méthanisation.

L'étape d'évacuation de la biomasse solide digérée résiduelle peut être effectuée par le biais du moyen de préhension apte à saisir la biomasse solide digérée résiduelle après méthanisation.

Une telle étape présente l'avantage de pouvoir se dérouler en toute sécurité après la vidange aérobie du digestat liquide, en absence de contact avec le biogaz.

Comme indiqué précédemment, l'unité de digestion principale peut être composée d'un ou plusieurs ouvrages.

D'autres avantages et caractéristiques de l'invention apparaîtront à l'examen de la description détaillée d'un mode de réalisation de l'invention nullement limitatif, et des dessins annexés, sur lesquels :

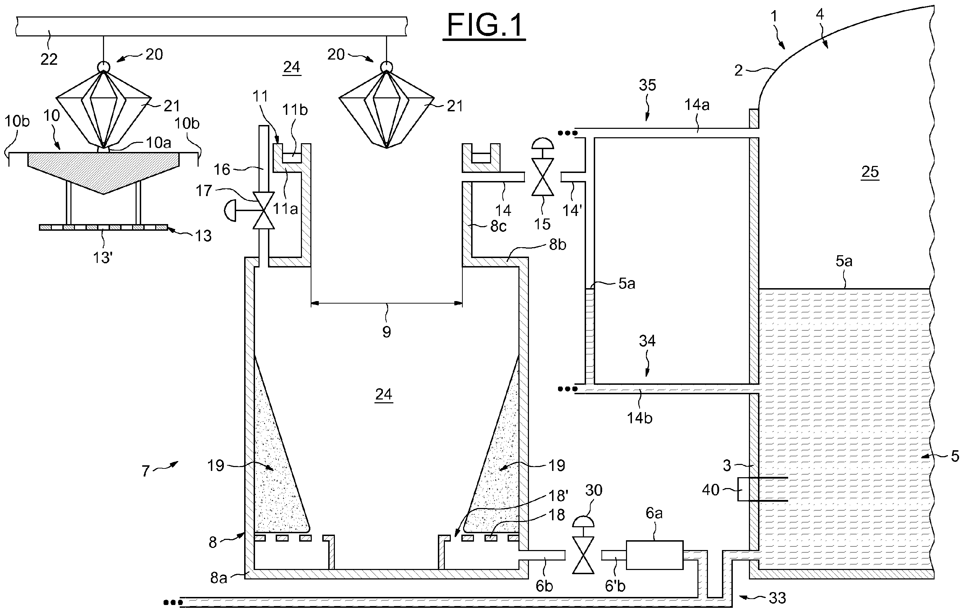

- la figure 1 représente schématiquement une vue en coupe du digesteur, selon l'invention, relié à une unité centrale de stockage de digestat liquide et de digestion complémentaire et d'une unité centrale de stockage de biogaz constituées d'une seule et même unité,

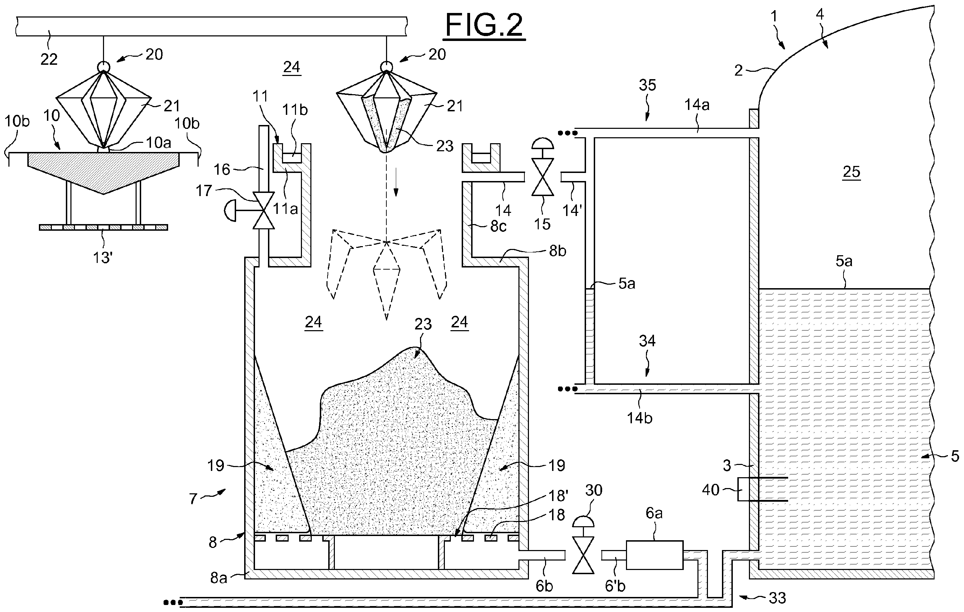

- les figures 2 à 9 représentent schématiquement des vues en coupe d'un digesteur anaérobie de biomasse solide, au cours des différentes étapes du procédé de production de biogaz, directement alimenté en digestat liquide en provenance d'une unité centrale de stockage de digestat liquide et de digestion complémentaire et d'une unité centrale de stockage de biogaz constituées d'une seule et même unité (liaison en parallèle),

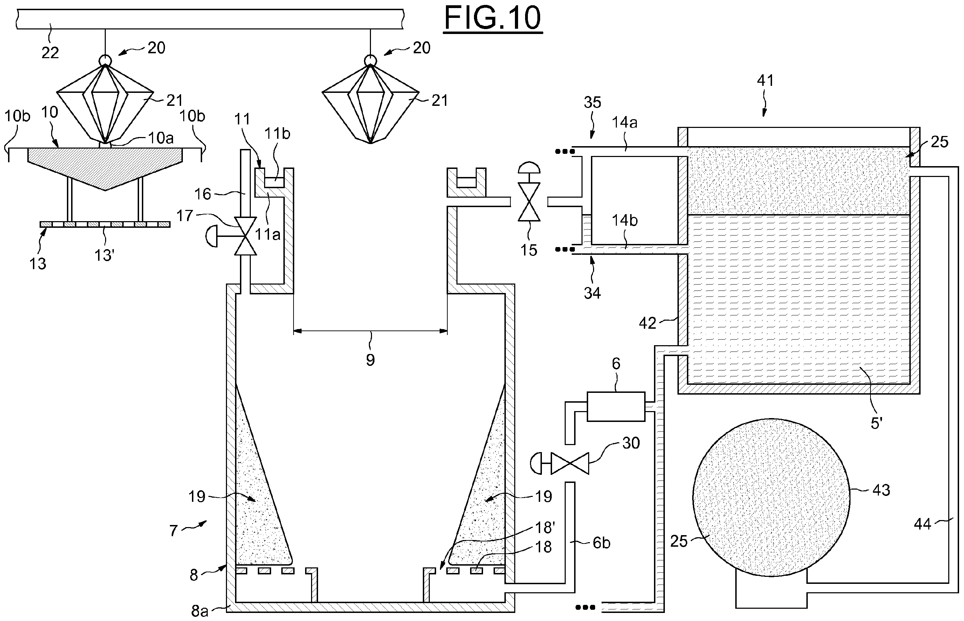

- la figure 10 représente schématiquement une vue du digesteur, selon l'invention, relié à une unité centrale de stockage de digestat liquide et de digestion complémentaire et une unité centrale de stockage de biogaz constituées de deux ouvrages distinctes l'une de l'autre,

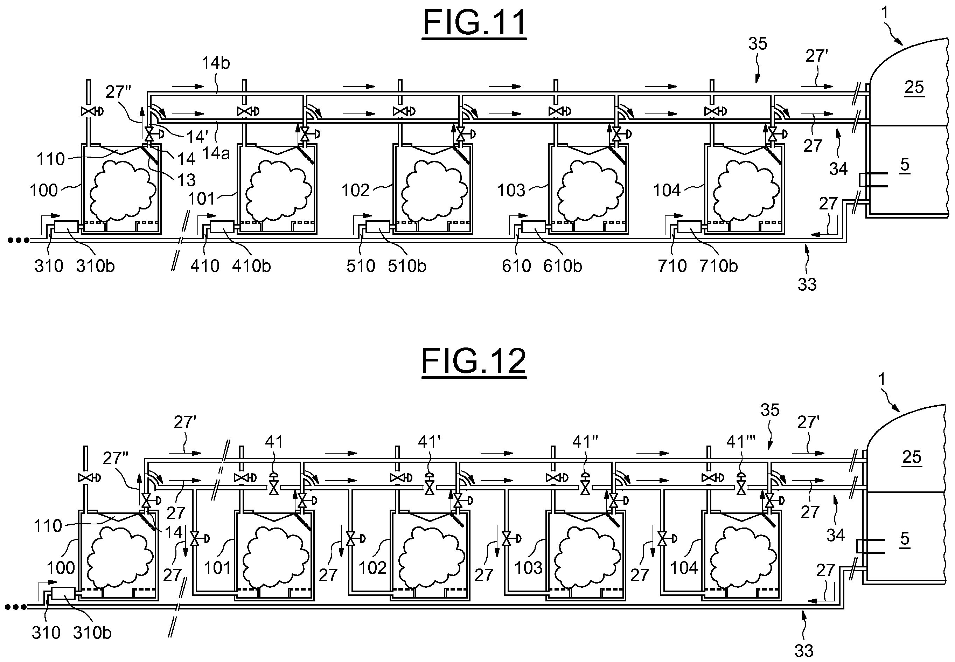

- la figure 11 représente schématiquement une vue en coupe d'une pluralité de digesteurs anaérobies de biomasse solide alimentés directement (liaison parallèle) en digestat liquide par une unité centrale de stockage de digestat liquide et de digestion complémentaire et d'une unité centrale de stockage de biogaz constitués d'une seule et même unité,
- la figure 12 représente schématiquement une vue en coupe d'une pluralité de digesteurs anaérobies de biomasse solide alimentés indirectement (liaison en série) en digestat liquide par une unité centrale de stockage de digestat liquide et de digestion complémentaire et d'une unité centrale de stockage de biogaz constitués d'une seule et même unité,

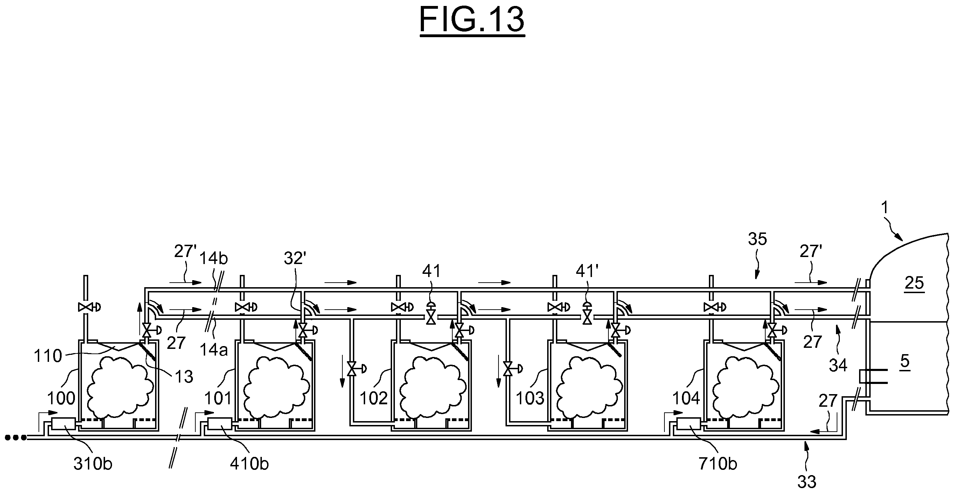

- la figure 13 représente schématiquement une vue en coupe d'une pluralité de digesteurs anaérobies de biomasse solide alimentés à la fois directement ou indirectement (liaison en série et en parallèle) en digestat liquide par une unité centrale de stockage de digestat liquide et de digestion complémentaire et d'une unité centrale de stockage de biogaz constitués d'une seule et même unité,

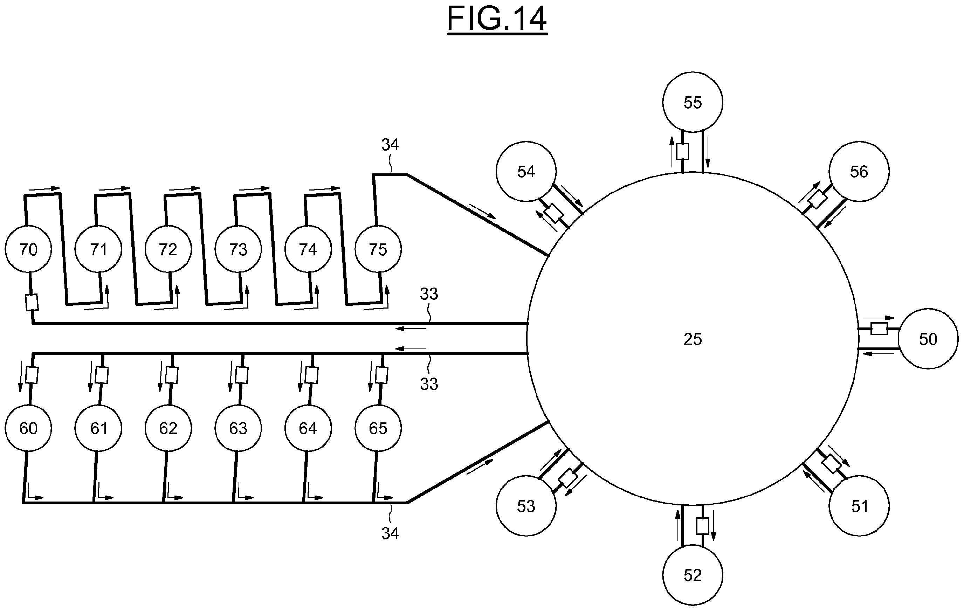

- la figure 14 illustre une vue de dessus d'un système de production de biogaz comprenant une pluralité de digesteurs anaérobies de biomasse solide et un réseau de circulation de digestat liquide.

Les figures 1 à 9 décrivent les différentes étapes successives d'un procédé de production de biogaz qui se déroulent au sein d'un digesteur anaérobie de biomasse dans le cas où ce dernier est directement alimenté en digestat liquide (liaison en parallèle) en provenance d'une unité centrale de stockage de digestat liquide et de digestion complémentaire et d'une unité de stockage de biogaz.

Les figures 1 à 9 se concentrent seulement sur une partie du système de production de biogaz.

Sur la figure 1 est représentée de manière schématique, une unité centrale de stockage de digestat liquide et de digestion complémentaire et une unité de stockage de biogaz constituant une seule et même unité, dénommé unité 1.

En d'autres termes, l'unité 1 constitue une seule et même unité centrale de stockage de digestat liquide, de biogaz et de digestion complémentaire.

Conformément à la figure 1, l'unité 1 est positionnée verticalement et s'étend de manière longitudinale.

L'unité 1 comprend une cuve 3, ayant une forme essentiellement cylindrique, remplie de digestat liquide 5, qui est surmontée d'une membrane étanche 2 constituant un espace de stockage de gaz 4, lequel est rempli de biogaz 25. La membrane 2 occupe donc la partie en hauteur de l'unité 1 formant ainsi un dôme contenant un ciel gazeux. La cuve 3 correspond à une réserve de digestat liquide 5.

Autrement dit, la réserve de digestat liquide 5 est surmontée par un espace de stockage de biogaz 25.

En variante, la cuve 3 peut présenter une forme cubique ou parallélépipédique.

La cuve 3 peut être fabriquée à partir d'acier, de béton, de matière plastique, ou de tout autre matériau tandis que la membrane étanche 2 peut être réalisée en polymère, notamment en polyéthylène, polypropylène ou chlorure de polyvinyle.

En variante, la membrane étanche 2 peut être surmontée par au moins une membrane supplémentaire de manière à ce que de l'air soit pris en sandwich entre la membrane supplémentaire et la membrane étanche 2.

Le digesteur 7 selon l'invention comprend une cuve 8, de forme essentiellement cylindrique, dotée d'un orifice d'entrée 9 sur lequel un toit amovible 10 est apte à se refermer et/ou à s'ouvrir. En variante, la cuve 8 peut présenter une forme essentiellement cubique ou parallélépipédique. Conformément à la figure 1, le digesteur 7 est positionné verticalement et s'étend de manière longitudinale

Plus précisément, selon le mode de réalisation représenté sur la figure 1, la cuve 8 présente une base 8a de forme essentiellement cylindrique, un col 8c et un épaulement 8b radial reliant la base 8a et le col 8c. Le col 8c forme ainsi un goulot d'étranglement. L'orifice d'entrée 9 de la cuve 8 présente ainsi une surface égale à la surface délimitée par le col 8c. Conformément à la figure 1, l'épaulement 8b est droit et horizontal mais peut également présenter une forme droite inclinée ou arrondie.

Selon un autre mode de réalisation la cuve 8 est essentiellement cylindrique et ne comprend pas de col 8c, ni d'épaulement radial 8b. Les digesteurs anaérobies de biomasse solide tels que représentés sur les figures 11, 12 et 13 fournissent une illustration d'un tel mode de réalisation.

La cuve 8 comprend en outre, sur la partie inférieure du digesteur 7, notamment au niveau de la base 8a, une tuyauterie 6b destinée à alimenter la cuve 8 en digestat liquide lors d'une étape de remplissage en digestat liquide.

La tuyauterie 6b comporte en outre une vanne 30, destinée à obturer ou permettre le passage du digestat liquide entre l'extérieur et l'intérieur du digesteur 7.

La tuyauterie 6b est montée sur un circuit d'alimentation 6'b en digestat liquide.

Le circuit d'alimentation 6'b est muni d'une pompe 6a qui est apte à faire circuler le digestat liquide 5 provenant de la cuve 3 de l'unité 1 vers la cuve 8 du digesteur 7.

Le circuit d'alimentation 6'b est relié à l'unité 1 par le biais d'un réseau d'alimentation 33 en digestat liquide. Conformément à la figure 1, le circuit d'alimentation 6'b est piqué sur le réseau d'alimentation 33 en digestat liquide au voisinage de la pompe 6a.

Le circuit d'alimentation 6'b fait ainsi partie intégrante d'un réseau d'alimentation 33 en digestat liquide 5 qui relie l'unité 1 au digesteur anaérobie 7.

Le réseau d'alimentation 33 en digestat liquide 5 est ainsi composé d'une pluralité de circuits d'alimentation en digestat liquide 5, similaires ou identiques au circuit d'alimentation 6'b, et permet de relier l'unité 1 au digesteur anaérobie 7 mais également à d'autres digesteurs anaérobies de biomasse solide (représentés sur les figures 11 à 14). Ainsi le circuit d'alimentation 6'b constitue l'un des circuits d'alimentation appartenant au réseau d'alimentation 33.

La pompe 6a permet ainsi non seulement d'alimenter le digesteur 7 en digestat liquide 5 provenant de l'unité 1 par le biais du circuit d'alimentation 6'b mais également de vidanger le digestat liquide 5 se trouvant à l'intérieur de la cuve 8, par le biais du même circuit 6'b, vers l'unité 1. Dans le mode de réalisation illustré dans la figure 1, la tuyauterie 6b relie la cuve 3, remplie en digestat liquide 5, de l'unité 1, par le biais du réseau d'alimentation 33, à la cuve 8 du digesteur 7, qui est vide.

Ainsi la tuyauterie 6b correspond à la fois à une ouverture d'alimentation en digestat liquide, lors de l'étape de remplissage de la cuve 8 en digestat liquide 5, et une ouverture de vidange de digestat liquide lors des étapes vidanges anaérobies et aérobies.

La cuve 8 comprend également une gouttière ou goulotte 11 disposée au voisinage de l'extrémité libre du col 8c. La gouttière 11 peut être réalisée monobloc avec le col 8c ou rapporté sur celui-ci par tout moyen approprié. La gouttière 11 est orientée du côté opposé à l'espace intérieur délimité par la cuve 8. En particulier, la gouttière 11 est orientée vers le haut.

La gouttière 11 contient un liquide 11a, de préférence de l'eau, et forme ainsi un joint liquide apte à assurer l'étanchéité, c'est-à-dire à éviter les fuites de biogaz ou les entrées d'air dans le digesteur anaérobie 7 lorsque le toit 10 est fermé.

Le col 8c de la cuve 8 comporte également une conduite 14, positionnée en-dessous de la gouttière 11, dont l'extrémité libre débouche dans la cuve 8. En particulier, la gouttière 11 est située sur la conduite 14 de manière à reposer sur celle-ci.

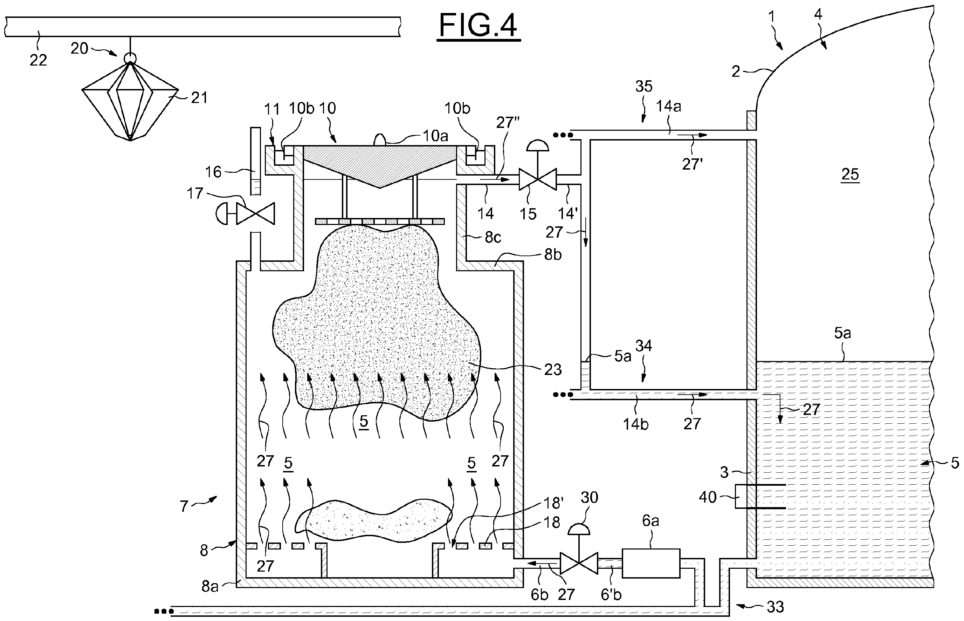

La conduite 14 est munie d'une vanne 15 qui est montée sur un circuit 14', situé en aval de la vanne 15, lequel circuit 14' se divise à son extrémité en deux circuits distincts 14a et 14b qui sont reliés à leur extrémité à l'unité 1. En particulier, la conduite 14' se divise à son extrémité en deux circuits distincts 14a et 14b en aval de la vanne 15. La conduite 14 permet de récupérer un fluide, formé lors de l'étape de digestion (décrite dans la figure 4), et la vanne 15 permet d'obturer ou de permettre la circulation du fluide au sein du circuit 14'. En particulier, la conduite 14 permet d'évacuer un mélange de digestat liquide et de biogaz, au cours de l'étape de digestion, vers le circuit 14'. La conduite 14 constitue une ouverture commune à l'évacuation du digestat liquide et du biogaz et le circuit 14' constitue un circuit commun d'évacuation de digestat liquide et de biogaz.

Le circuit 14a permet d'évacuer du biogaz vers l'espace de stockage rempli de biogaz 25 de l'unité 1 et le circuit d'évacuation 14b du digestat liquide vers la cuve 3, remplie de digestat liquide 5, de l'unité 1. Le circuit 14a d'évacuation du biogaz est ainsi positionné au-dessus du circuit 14b d'évacuation du digestat liquide ce qui facilite le transfert des différents fluides vers l'unité 1.

Selon un mode de réalisation, le circuit 14b peut comprendre une pompe de circulation (non représenté sur la figure 1) afin de faciliter l'acheminement du digestat liquide.

En particulier, le circuit 14b est positionné, en son extrémité, sous le niveau 5a du digestat liquide 5 remplissant la cuve 3 de l'unité 1, de telle sorte que le circuit 14b contienne toujours du digestat liquide en son extrémité.

Le circuit d'évacuation 14' de biogaz 25 et de digestat liquide 5, relié à la conduite 14, est un circuit appartenant à un réseau d'évacuation 34 de digestat liquide 5 et un réseau d'évacuation 35 de biogaz 25. Autrement dit, le circuit d'évacuation 14' est commun au réseau d'évacuation 34 de digestat liquide 5 et au réseau d'évacuation 35 de biogaz 25.

En particulier, le circuit 14a de biogaz appartient au réseau d'évacuation 35 de biogaz 25 et le circuit d'évacuation 14b de digestat liquide 5 appartient au réseau d'évacuation 34 de digestat liquide 5.

Le réseau d'évacuation 34 de digestat liquide 5 comporte donc une pluralité de circuits d'évacuation de digestat liquide, identiques ou similaire au circuit d'évacuation 14b et au circuit 14', et le réseau d'évacuation 35 de biogaz 25 comporte une pluralité de circuits d'évacuation de biogaz, identiques ou similaire au circuit d'évacuation 14a et au circuit 14' ; le circuit d'évacuation 14' de digestat liquide et de biogaz étant commun aux deux réseaux.

Le réseau d'évacuation 34 de digestat liquide 5 et le réseau d'évacuation 35 de biogaz 25 relient l'unité 1 au digesteur anaérobie 7 mais également à d'autres digesteurs anaérobies de biomasse solide (représentés sur les figures 11 à 14).

Plus généralement, le réseau d'alimentation 33 en digestat liquide 5, le réseau d'évacuation 34 de digestat liquide 5 et le réseau d'évacuation 35 de biogaz 25 permettent de relier l'unité 1 à d'autres digesteurs anaérobies de biomasse solide (non représentés sur la figure 1).

La cuve 8 comporte également une tuyauterie 16 qui est en saillie vers l'extérieur par rapport à l'épaulement 8b. La tuyauterie 16 est ouverte vers l'extérieur ce qui permet d'acheminer l'air à l'intérieur de la cuve 8 du digesteur 7 ou de l'évacuer vers l'extérieur de la cuve 8. La vanne 17, montée sur la tuyauterie 16, permet de rendre possible ou d'empêcher l'introduction ou l'évacuation de l'air à l'intérieur de la cuve 8. La tuyauterie 16 se trouve ainsi située sur la partie supérieure du digesteur 7.

La tuyauterie 16 correspond ainsi à une ouverture d'introduction et/ou d'évacuation d'air apte à permettre l'introduction et/ou l'évacuation d'air dans le digesteur.

Le digesteur 7 est ainsi relié à l'unité 1, d'une part, par le biais d'une ouverture 6b commune d'alimentation et de vidange de digestat liquide reliée au circuit d'alimentation et de vidange 6'b de digestat liquide et, d'autre part, par le biais d'une ouverture 14 commune d'évacuation de digestat liquide et de biogaz reliée à au moins un circuit d'évacuation de digestat liquide et de biogaz.

Le digesteur anaérobie 7 comprend également un élément de séparation perforé 18 situé en amont de la tuyauterie de vidange 6b.

L'élément de séparation perforé 18 est fixé sur le fond intérieur de la cuve 8 et occupe partiellement ou totalement le fond du digesteur 7. Ainsi l'élément de séparation perforé de vidange du digestat liquide18 peut correspondre à une couronne perforée occupant partiellement le fond de la cuve 8.

De manière alternative, l'élément de séparation perforé 18 peut correspondre à un élément vertical situé en surépaisseur de la virole ou de la partie du mur vertical de la cuve 8. L'élément de séparation perforé de vidange du digestat liquide 18 peut également correspondre à tout autre type d'élément perforé situé en amont de la tuyauterie de vidange 6b, lors des opération de vidange aérobie ou anaérobie du digestat liquide de la cuve 8 du digesteur 7 vers la cuve 3 de l'unité 1.

L'élément de séparation perforé 18 comprend une pluralité d'interstices 18' afin de permettre la circulation du digestat liquide lors des étapes de vidanges anaérobies et aérobies du digesteur 1 tout en retenant la biomasse solide dans la cuve 8.

Un grappin 20 est positionné au-dessus de l'orifice d'entrée 9 de la cuve 8 et est suspendu sur un pont roulant 22 par l'intermédiaire d'un câble ou d'un filin pouvant être en acier. Le grappin 20, tel que représenté, comporte une pluralité de crochets 21 et est piloté à partir du pont roulant 22. En variante, le grappin 20 correspond à une benne ayant une pluralité de godets.

Conformément à la figure 1, le grappin 20 peut saisir le toit amovible 10 de la cuve 8.

Le toit amovible 10 présente une surface sensiblement horizontale pourvue d'un élément 10a facilitant la préhension du toit 10 par le grappin 20, telle qu'une anse, une poignée ou une manicle. Le toit amovible 10 est ainsi placé sur l'orifice d'entrée 9 de la cuve 8 ou déplacé de l'orifice d'entrée 9 de la cuve 8 par l'intermédiaire du grappin 20.

Le toit amovible 10 présente des rebords 10b, conformés de manière à venir s'engager dans le liquide 11a de la gouttière 11 lorsque le toit 10 est installé sur l'orifice d'entrée 9 de la cuve 8.

En particulier, les rebords 10b comprennent une partie horizontale et une partie verticale qui prolonge vers le bas la partie horizontale.

Le toit amovible 10 est surmontée vers le bas d'un élément de séparation perforé 13 comprenant une pluralité d'interstices 13' afin de permettre l'évacuation du digestat liquide au cours de l'étape de digestion de la biomasse solide tout en retenant la biomasse solide dans la cuve 8.

L'élément de séparation perforé 13 est fixé sur le toit 10 amovible de manière à ce qu'il se positionne en amont de la tuyauterie 14 lorsque le toit 10 amovible est monté l'intérieur de l'orifice d'entrée 9 de la cuve 8.

En particulier, l'élément de séparation perforé 13 est une plaque ou une grille perforée.

Selon un autre mode de réalisation, l'élément de séparation perforé 13 n'est pas fixé sur le toit 10 amovible, mais est fixé dans le col 8c de la cuve 8, sous le niveau de la tuyauterie 14, de manière à ce qu'il se positionne en amont de la tuyauterie 14 lors des opérations d'évacuation de digestat liquide. Dans ce cas là, l'élément de séparation perforé 13 est amovible et doit être retiré pendant les opérations de chargement de la biomasse solide ou de déchargement de la biomasse solide digérée résiduelle.

Selon un autre mode de réalisation, l'élément de séparation perforé 13 n'est pas fixé sur le toit 10 amovible ni dans le col 8c, en particulier quand la cuve 8 n'inclut pas de col 8c, mais est fixé en tout autre lieu de la virole de la cuve 8, dans tous les cas en amont de l'ouverture d'évacuation du digestat liquide 14. Les digesteurs anaérobies de biomasse solide tels que représentés sur les figures 11, 12 et 13 fournissent une illustration d'un tel mode de réalisation.

Le système de production tel que représenté comprend également un dispositif de chauffage 40 du digestat liquide.

Le dispositif de chauffage 40 est plus particulièrement positionné au niveau de la cuve 3 de l'unité 1 de manière à chauffer le digestat liquide 27 de telle sorte que le digestat liquide contenu dans l'unité centrale 1 ait une température constante généralement comprise entre 45 et 55°C pour un régime thermophile et entre 35 et 40°C pour un régime mésophile.

En outre, le digesteur 7 et l'unité 1 sont calorifugés.

Sur la figure 2 est représentée schématiquement l'étape de remplissage de la cuve 8 du digesteur 7 par de la biomasse solide 23, en particulier fibreuse et/ou hétérogène, à l'aide du grappin 20.

Au cours de cette étape, le toit amovible 10 n'est pas montée sur l'orifice d'entrée 9 et l'élément de séparation perforé 13 n'est pas non plus installé dans le col 8c, c'est-à-dire que l'extrémité du col 8c est ouverte.

Le grappin 20 permet de saisir la biomasse solide 23 stockée à l'extérieur du digesteur 7 afin de la déposer à l'intérieur de la cuve 8 du digesteur 7. En d'autres termes, le grappin 20 permet d'alimenter, par le haut, la cuve 8 du digesteur 7, en biomasse solide 23, à travers l'orifice d'entrée 9 qui est situé au niveau de la surface supérieure 12 de la cuve 8 du digesteur 7. Ainsi l'orifice d'entrée 9 constitue une zone d'admission de la biomasse solide 23.

Le grappin 20 permet de saisir la biomasse solide 23 contenant éventuellement des éléments indésirables, tels que des cailloux, du gravier, du sable, des barbelés, des ficelles, des pièces métalliques provenant d'outils agricoles ou encore des sacs plastiques, sans avoir besoin d'effectuer d'opération de tri préalable, ni de broyage, ni de dilution de la biomasse solide dans un liquide.

En fonction de son taux de matière sèche, une fraction liquide de la biomasse 23 peut éventuellement couler du grappin au moment de l'étape d'alimentation de la cuve 8.

Le grappin 20 a également pour fonction d'ouvrir le toit amovible 10 pour le déposer à côté de la cuve 8 ou plus loin, et à le refermer par la suite.

Selon un mode de réalisation, lorsque n'est pas fixé sur le toit 10 amovible, mais est fixé dans le col 8c de la cuve 8, sous le niveau de de la tuyauterie 14, le grappin 20 peut également avoir pour fonction de saisir l'élément de séparation perforé 13, après qu'il a été décroché du col 8c, pour le déposer à côté de la cuve 8 ou plus loin, et à le remettre dans le col 8c de la cuve 8 par la suite.

La biomasse solide 23 se trouve alors à l'intérieur de la cuve 8 et repose sur le fond et/ou tout ou partie de l'élément de séparation perforé de vidange du digestat liquide 18.

Les interstices 18' de l'élément de séparation perforé de vidange du digestat liquide 18 présentent une taille suffisamment faible pour éviter que la biomasse 23 ne puisse pénétrer dans la tuyauterie 6b lors des étapes de vidange anaérobies et aérobies de la cuve 8.

L'étape d'alimentation est arrêtée à partir du moment où la quantité en biomasse solide 23 introduite est jugée suffisante pour démarrer la méthanisation.

Lorsque l'étape de remplissage de la cuve 8 par de la biomasse solide 23 est terminée, le toit amovible 10 est montée à l'intérieur de la surface délimitée par le col 8c de manière à recouvrir complètement de façon étanche l'orifice d'entrée 9.

En particulier, le grappin 20 permet de positionner le toit amovible 10 à l'intérieur de la surface délimitée par le col 8c et d'engager les rebords 10b dans le liquide 11a du joint liquide 11.

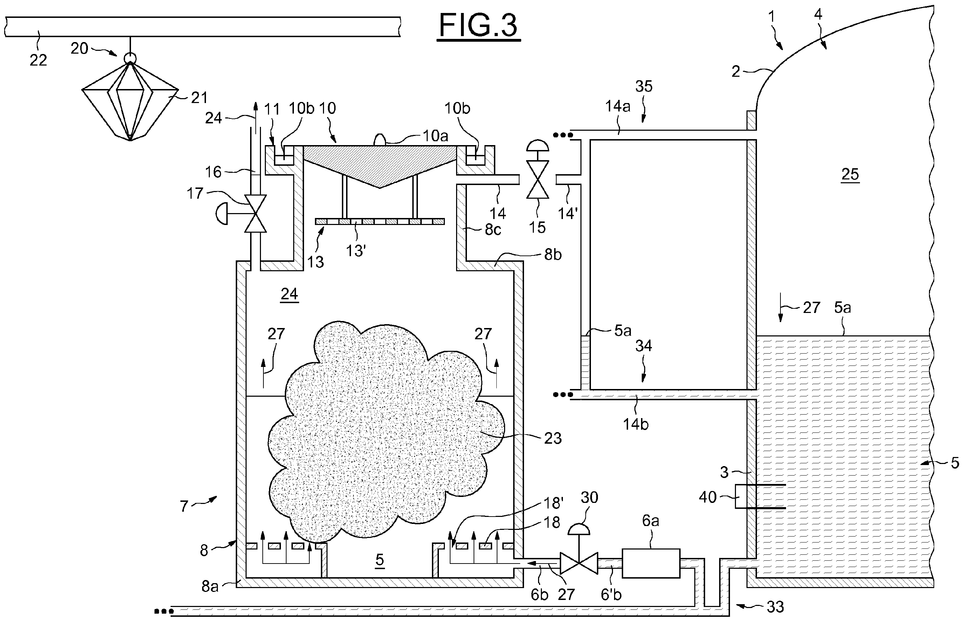

Ainsi la figure 3 illustre la cuve 8 du digesteur 7, après fermeture du toit amovible 10, qui est remplie avec du digestat liquide 5, acheminé par le biais de la pompe d'alimentation 6a, en provenance de la cuve 3 de l'unité 1. En particulier, sur la figure 3, le digestat liquide 5 est acheminé au niveau de la partie inférieure de la cuve 8 sous l'élément perforé 18 et circule à travers les interstices 18' de l'élément de séparation 18.

Dans un autre mode de réalisation, la cuve 8 peut être remplie avec du digestat liquide 5 qui ne passe pas à travers l'élément perforé inférieur 18.

De manière alternative, le digestat liquide 5 peut être aussi acheminé par une entrée située en tout autre endroit de la cuve 8.

Au cours de cette étape de remplissage de la cuve 8 par du digestat liquide 5, la vanne 17 est laissée en position ouverte de manière à ce que l'air, présent dans la cuve 8, soit progressivement évacué du digesteur 7 par l'intermédiaire de la tuyauterie 16. Sur la figure 3 est représenté schématiquement, l'air 24 qui s'échappe de la tuyauterie 16 pendant le remplissage en digestat liquide 5 de la cuve 8. La vanne 15 est fermée de manière à ce que l'air 24 ne circule pas vers l'unité 1 et de manière à ce que du biogaz 25 contenu dans la cuve 3 ne puisse pas pénétrer dans le digesteur 7.

De même, la vanne 30 est en position ouverte de manière à ce que le digestat liquide 5 alimente la cuve 8 du digesteur 7.

La biomasse solide 23, et éventuellement la biomasse solide digérée résiduelle 19 issue d'un précédent cycle de méthanisation, se retrouve alors immergée dans le digestat liquide 5 à l'intérieur de la cuve 8. Dans le mode de réalisation illustré dans la figure 3, la biomasse solide 23 et les résidus de biomasse digérée 19 se retrouvent mélangées entre eux.

Le niveau du digestat liquide 5 dans la cuve 3 du digesteur principal 1 diminue tandis qu'il augmente à l'intérieur de la cuve 8 chassant l'air, se trouvant à l'intérieur de la cuve 8, qui s'échappe à travers la tuyauterie 16. L'étape de remplissage sera considérée comme étant terminée lorsque le niveau du digestat liquide 5 aura atteint et légèrement dépassé vers le haut le niveau de la vanne 17, et que la totalité de l'air contenu dans la cuve 8 aura été évacuée. Sur la figure 3 ce niveau est aussi celui de l'ouverture d'évacuation de digestat liquide.

Sur la figure 3 est représenté, d'une part, le sens de circulation 27 du digestat liquide 5 à l'intérieur de la cuve 8 du digesteur 7 qui reflète l'augmentation de niveau dans la cuve 8 et, d'autre part, le sens de circulation 27 qui illustre la baisse de niveau du digestat liquide 5 à l'intérieur de l'unité de digestion principale 1.

De plus, le sens de circulation 27 du digestat liquide 5 est représenté à l'intérieur du circuit 6'b et de la tuyauterie 6b.

Conformément à la figure 4, une fois la cuve 8 remplie par le digestat liquide 5, la vanne 17 est fermée de manière à placer le digesteur 7 dans des conditions anaérobies, c'est-à-dire à empêcher toute introduction d'air dans la cuve 8, tandis que la vanne 15 est ouverte afin de permettre l'évacuation du mélange biogaz et digestat liquide.

La figure 4 représente donc l'étape de digestion de la biomasse 23 dans des conditions anaérobies. Au cours de cette étape, du biogaz se forme au sein de la cuve 8.

En particulier, lorsque le niveau du digestat liquide 27 a atteint le niveau de débordement, qui se situe légèrement au-dessus du niveau de la vanne 17, et que le digestat liquide 5 commence à déborder dans l'ouverture 14, la vanne 17 est alors fermée, afin de placer le digesteur 1 dans des conditions anaérobies.

La figure 4 représente donc l'étape de digestion de la biomasse 23 dans des conditions anaérobies. Au cours de cette étape, du biogaz se forme au sein de la cuve 8.

Une percolation du digestat liquide 5 à travers la biomasse 23 est mise en œuvre par le pompage du digestat liquide 5 en provenance de l'unité 1. La percolation se déroule à travers la biomasse solide 23 de manière à ce que la plus grande partie possible de la biomasse solide 23 soit mise en contact intime avec les bactéries contenues dans le digestat liquide 5 et puisse ainsi réagir au cours de la digestion. Dans la figure 4 est représenté schématiquement le flux de digestat liquide 5 circulant, par percolation verticale ascendante, à travers l'ensemble de la biomasse solide 23.

Conformément à la figure 4, de préférence, l'alimentation en digestat liquide 5 continue au cours de toute l'étape de digestion afin que le digestat liquide 5 continue de circuler à travers la biomasse solide 23, ce qui diminue le risque de survenue de réactions chimiques ou biologiques ponctuelles indésirables en certains endroits du tas de biomasse solide 23, tel que l'acidose par exemple, lesquelles pourraient altérer la qualité de la flore bactérienne.

Cependant, l'alimentation en digestat liquide 5 peut être, dans certains cas, temporairement ou définitivement interrompue, en particulier vers la fin du cycle de méthanisation, moment où les réactions chimiques et biologiques sont moins virulentes.

Au cours de cette étape, un processus de diffusion du digestat liquide 5 dans la biomasse solide 23 se produit. Un tel processus de diffusion est favorisée, d'une part, par l'immersion de la biomasse solide 23 dans le digestat liquide 5 et, d'autre part, par la pression du digestat liquide 5 sur la biomasse solide 23 qui résulte de l'action de la pompe 6a.

Dans le mode de réalisation de la figure 4, la biomasse solide 23 se désagrège au cours de la percolation du digestat liquide 5 à travers la biomasse solide 23 et de la diffusion du digestat liquide 5 dans la biomasse solide 23.

La biomasse solide 23, selon sa nature, peut flotter et remonter dans le digesteur au cours de l'étape de digestion de manière à venir s'appuyer contre l'élément de séparation 13.

Le biogaz 25 et le digestat liquide 5 circulent à travers les interstices 13' de l'élément de séparation perforé 13 tandis que la biomasse solide 23 reste immergée dans la cuve 8 et est retenue par l'élément de séparation 13.

Le mélange de biogaz 25 et de digestat liquide 5, circulant à travers l'élément de séparation perforé d'évacuation du digestat liquide 13, est représenté schématiquement sur la figure 4.

Le biogaz 25 et le digestat liquide 5 sont ensuite acheminés ensemble dans la tuyauterie 14 et circulent dans le circuit d'évacuation 14' jusqu'à la portion où ledit circuit d'évacuation 14' se divise en deux circuits distincts 14a et 14b.

Le sens de circulation 27' du biogaz 25 et du digestat liquide 5 a été représenté dans la tuyauterie 14 et le circuit d'évacuation 14'.

Le biogaz 25 et le digestat liquide 5 sont évacués dans le circuit d'évacuation 14' pour être ensuite séparés au niveau de son extrémité dans le circuit 14a d'évacuation de biogaz et le circuit 14b d'évacuation de digestat liquide.

Sur la figure 4 est représenté le niveau 5a du digestat liquide 5 dans le circuit 14b qui se retrouve à la même hauteur que le niveau du digestat liquide 5 dans l'unité 1.

Le digestat liquide 5 se retrouve acheminé dans un circuit d'évacuation 14b correspondant à une conduite de débordement du digestat liquide. Le digestat liquide 5 présent dans le circuit 14b est acheminé jusqu'à la cuve 3, remplie de digestat liquide 5, l'unité 1. Dans la figure 4, le digestat liquide 5 injecté dans la cuve 8 du digesteur 7, par le biais de la pompe 6, est recirculé par débordement, au moyen du circuit d'évacuation 14b, dans la cuve 3 de l'unité 1. Le sens de circulation du digestat liquide 5 est représenté par les flèches 27.

Optionnellement, la présence d'une pompe de circulation (non représentée sur la figure 4) montée sur le circuit 14b permet d'aspirer le digestat liquide 5 à travers l'élément de séparation perforé 13.

Le biogaz 25 est quant à lui acheminé dans le circuit 14a jusqu'au digesteur principal 1, en particulier à un niveau proche de la membrane étanche 2, lequel niveau est de préférence supérieur au niveau maximal de stockage du digestat liquide 5 dans la cuve 3 de l'unité 1.

L'étape de digestion permet donc de produire du biogaz sans avoir à mettre en œuvre une étape de brassage de la biomasse solide 24 ce qui permet de diminuer les investissements en matériels de brassage et d'agitation et de réduire les dépenses énergétiques et de maintenance généralement afférentes à cette étape.

En variante, les ouvertures d'évacuation du digestat liquide et du biogaz peuvent être distinctes, et la séparation du biogaz 25 et du digestat liquide 27 peut s'effectuer directement dans la cuve 8 du digesteur 7.

Durant l'étape de digestion, la biomasse solide 23 est immergée dans le digestat liquide 5 et peut en partie flotter en haut du digesteur anaérobie 1, se trouver en partie en suspension au milieu du digesteur anaérobie 1 ou en partie décanter et se trouver en bas du réacteur anaérobie 1.

Le biogaz 25 est quant à lui acheminé dans le circuit 14a jusqu'à l'unité 1, en particulier à un niveau proche de la membrane étanche 2, lequel niveau est de préférence supérieur au niveau maximal de stockage du digestat liquide 5 dans la cuve 3 de l'unité 1.

Ainsi l'élément de séparation perforé 13 est localisé en amont de la tuyauterie 14 qui est située au niveau de la partie supérieure de la cuve 8 du digesteur 7.

L'étape de digestion, permet de produire du biogaz sans avoir à mettre nécessairement en œuvre une étape de brassage de la biomasse solide 23 ce qui permet de diminuer les investissements en matériels de brassage et d'agitation et de réduire les dépenses énergétiques et de maintenance généralement afférentes à cette étape.

Conformément à la figure 4, le joint liquide 11 permet d'assurer une meilleure étanchéité du toit 10 et d'éviter les fuites éventuelles de biogaz, au cours de l'étape de digestion, susceptibles de survenir au niveau des rebords 10b du toit amovible 10.

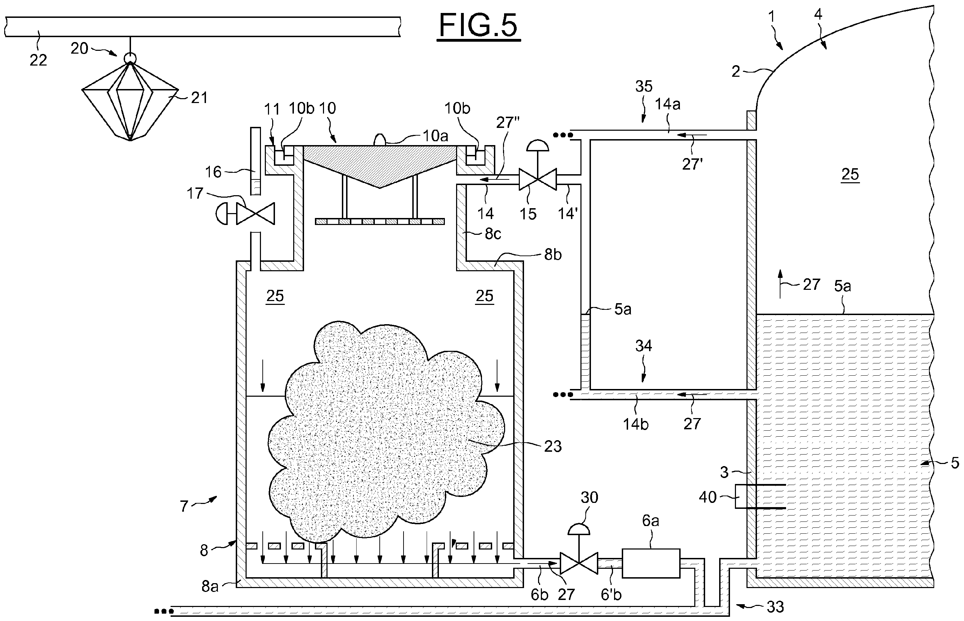

Sur la figure 5 est représentée schématiquement l'étape de vidange anaérobie du digestat liquide 5 de la cuve 8 du digesteur 7, vers la cuve 3 de l'unité de digestion principale 1, en vidangeant le digestat liquide 5 par l'intermédiaire de la pompe 6a et en conservant la vanne 17 en position fermée tandis que la vanne 15 est en position ouverte.

L'étape de vidange anaérobie permet, par le tassement au fond du digesteur 7 de la biomasse solide 23 partiellement digérée suite à l'étape de digestion et de diffusion, de supprimer les éventuels chemins préférentiels de percolation ainsi que les éventuelles poches de biogaz, bloquées dans la biomasse 23. Ainsi le biogaz 25, se trouvant dans le circuit 14a, circule depuis l'unité 1 en direction de l'intérieur de la cuve 8 du digesteur 7 en passant à travers l'élément de séparation perforé 13. Le biogaz vient ainsi remplir l'espace laissé vacant par la vidange du digestat liquide 5. Au cours de cette étape de vidange, la biomasse solide 23 redescend vers le fond de la cuve 8 en direction de l'élément de séparation perforé 18, et vient finalement se déposer sur le fond de la cuve 8 et/ou sur l'élément de séparation perforé de vidange du digestat liquide 18.

Sur la figure 5 est représenté le sens de circulation 27" du mélange de biogaz 25 et de digestat liquide 5 dans le circuit 14', et le sens de circulation 27' du biogaz 25 dans le circuit 14a en direction de la cuve 8 ainsi que le sens de circulation 27 du digestat liquide 5 en direction de la cuve 3 de l'unité 1.

L'élément de séparation perforé 18 présente l'avantage de laisser passer le digestat liquide 5 au moment de la vidange anaérobie en retenant dans la cuve 8 la biomasse solide 23 partiellement digéré. L'élément de séparation perforé 18 permet donc de séparer le digestat liquide 5, en le laissant circuler, et la biomasse solide 23 ce qui permet de ne pas risquer d'obstruer la pompe d'alimentation et de vidange 6a et de ne pas introduire du digestat solide 23 dans l'unité 1.

Conformément à la figure 5, le niveau du digestat liquide 27 augmente dans l'unité 1 au moment de l'étape de vidange anaérobie.

En variante, l'ouverture de vidange de digestat liquide peut être distincte de l'ouverture d'alimentation en digestat liquide. Dans ce cas, l'ouverture de vidange de digestat liquide peut être connectée à un circuit de vidange qui est relié à une fosse de relevage.

Conformément à cette variante, le digestat liquide s'écoule de manière gravitaire vers la fosse de relevage depuis laquelle le digestat liquide 27 est ensuite pompé une nouvelle fois dans l'unité 1.

En outre, le circuit peut être équipé d'une pompe de vidange et la vidange de cuve 8 peut de cette manière être réalisée par pompage.

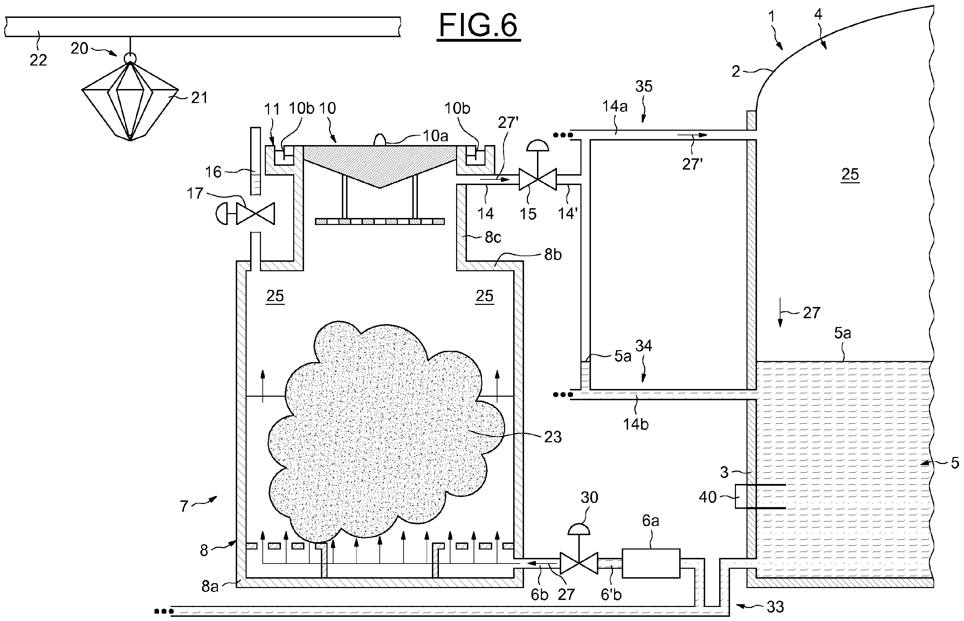

Après la vidange anaérobie de la cuve 8, une nouvelle étape de remplissage de la cuve 8 avec du digestat liquide 5 est mise en œuvre comme représenté sur la figure 6. L'étape de remplissage est identique à la précédente étape de remplissage à l'exception du fait que la vanne 17 est en position fermée et que la vanne 15 est en position ouverte, de telle sorte que le biogaz 25 qui a été introduit dans la cuve 8 lors de l'étape de vidange anaérobie soit renvoyé dans le ciel gazeux de la cuve 3 lors de l'opération de re-remplissage de digestat liquide.

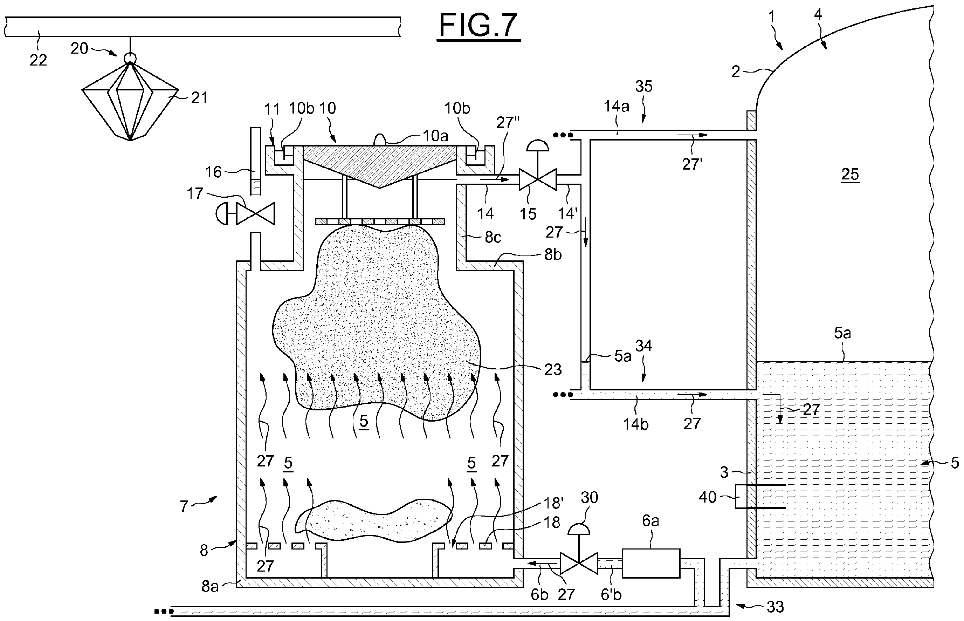

Conformément à la figure 7, une fois l'étape de remplissage terminée, l'étape de digestion de la biomasse solide 23 est une nouvelle fois mise en œuvre afin de continuer la production de biogaz 25.

De la même façon que précédemment, le biogaz 25, issu de l'étape de digestion, est principalement acheminé par le biais du circuit d'évacuation 14a jusqu'à l'unité 1.

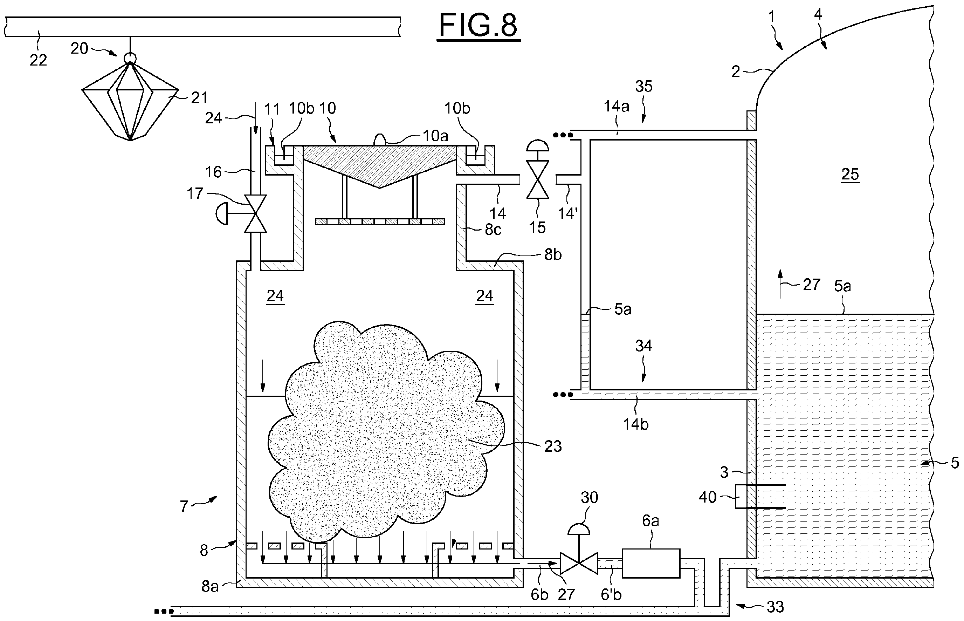

Une fois l'étape de digestion terminée, c'est-à-dire en fin de cycle au bout d'un délai généralement compris entre 20 et 80 jours, une étape de vidange aérobie du digesteur 7 est mise en œuvre comme illustré sur la figure 8.

L'étape de vidange aérobie est mise en œuvre en fermant la vanne 15 afin d'empêcher toute introduction de biogaz 25 à l'intérieur de la cuve 8, et en ouvrant la vanne 17 afin d'introduire l'air 24 à l'intérieur de la cuve 8 de digestion en remplacement du digestat liquide 5, qui est vidangé par la pompe 6a à travers la tuyauterie 6b après ouverture de la vanne 30, à travers l'élément de séparation perforé 18, vers la cuve 3 de l'unité 1.

Cette étape permet de vidanger le digestat liquide de la cuve 8 de digestion en évitant la présence de biogaz dans la cuve 8, ce qui permet de minimiser les fuites/rejets de biogaz dans l'atmosphère au moment de la mise en œuvre ultérieure de l'étape d'évacuation de la biomasse solide 19 résiduelle après digestion, c'est-à-dire le digestat solide.

Sur cette figure, le niveau du digestat liquide 5 dans la cuve 8 diminue et l'air 24 s'introduit progressivement à l'intérieur de la cuve 8 du digesteur 7 en comblant l'espace laissé vacant par la vidange du digestat liquide 5.

En variante, l'ouverture de vidange de digestat liquide peut être distincte de l'ouverture d'alimentation en digestat liquide. Dans ce cas, l'ouverture de vidange de digestat liquide est connectée à un circuit de vidange qui peut être relié à une fosse de relevage.

Conformément à cette variante, au cours de l'étape de vidange aérobie, le digestat liquide s'écoule de manière gravitaire vers la fosse de relevage depuis laquelle le digestat liquide 5 peut éventuellement être pompé dans l'unité 1.

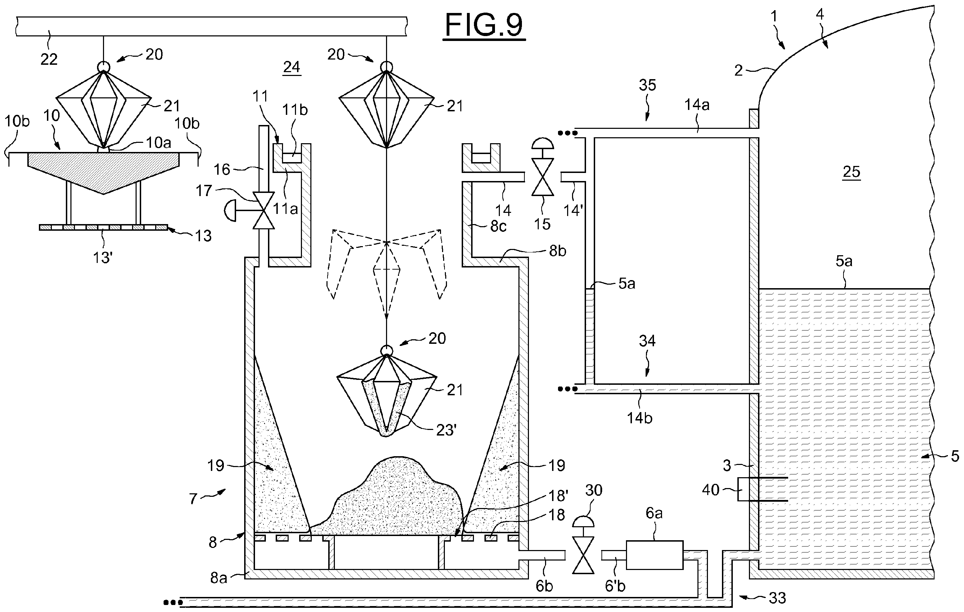

La figure 9 décrit l'étape d'évacuation de la biomasse solide digérée résiduelle 19, une fois la digestion terminée, par le grappin 20. En particulier, le grappin 20 saisit le toit amovible 10 pour le retirer de l'orifice d'entrée 9.

De cette manière, le grappin 20 permet de saisir la biomasse solide digérée résiduelle 19, une fois la digestion terminée, de manière à l'évacuer soit vers une zone de stockage, une zone de traitement ou un engin de transport.

Dans le mode de réalisation illustré sur la figure 9, le grappin 20 saisit la biomasse solide digérée résiduelle 23' dans la cuve 8 du digesteur 7 en laissant éventuellement subsister des résidus de biomasse solide digérée 19 au niveau des parois ou du fond de la cuve 8.

Des résidus de biomasse solide digérée 19 peuvent être laissés au niveau des parois, ou dans le fond de la cuve 8, ou encore sur l'élément de séparation inférieur 18, car ils peuvent constituer un bon substrat, très riche en bactéries qui seront susceptibles d'agir lors du prochain cycle de méthanisation.

Grâce à l'étape de vidange aérobie précédente, l'étape de récupération de la biomasse solide digérée résiduelle 19 après méthanisation, correspondant au digestat solide, peut se dérouler en toute sécurité en minimisant les risques de rejet de biogaz dans l'atmosphère ainsi que les risques de mise en contact avec le biogaz des personnes, le grappin et des équipements situés en dehors de la cuve 8 de digestion.

Ainsi l'orifice d'entrée 9 de la cuve 8 représente à la fois une zone d'admission de la biomasse solide 23 avant méthanisation ainsi qu'une zone d'évacuation de la biomasse solide digérée résiduelle 19 après méthanisation, appelée digestat solide

En d'autres termes, l'orifice d'entrée 9 de la cuve 8 constitue un espace d'introduction de la biomasse solide et d'évacuation de la biomasse solide digérée résiduelle.

Conformément au mode de réalisation illustré dans les figures 1 à 9, la zone d'admission de biomasse solide 23 correspond à la zone d'évacuation de la biomasse solide.

En variante, la zone d'admission de biomasse solide 23 peut être distincte de la zone d'évacuation de la biomasse solide digérée résiduelle 23'.

Selon un tel mode de réalisation, la zone d'évacuation de la biomasse solide peut ne pas être située sur la partie supérieure de la cuve 8 du digesteur 7.

Sur la figure 10 est représenté schématiquement une vue en coupe du digesteur, selon l'invention, relié à une unité centrale de stockage de digestat liquide et de digestion complémentaire et une unité centrale de stockage de biogaz constituées de deux ouvrages distinctes l'une de l'autre.

En particulier, l'unité centrale de stockage de digestat liquide et de digestion complémentaire et l'unité centrale de stockage de biogaz constituent deux unités distinctes composées chacune d'un ouvrage.

Conformément à cette figure, l'unité centrale 41 de stockage de digestat liquide et de digestion complémentaire comprend une cuve 42 contenant du digestat liquide 5 surmontée en surface par une faible quantité en biogaz 25 et l'unité centrale 43 de stockage de biogaz correspond à un gazomètre apte à stocker du biogaz 25.

Les deux unités 41 et 43 sont reliées entre elles par une tuyauterie 44.

Sur la figure 10, le circuit d'évacuation 14' de biogaz 25 et de digestat liquide 5 se divise en deux circuits distincts 14a et 14b qui sont reliés à l'unité centrale 41.

De la même façon que précédemment, d'une part, le circuit 14b est un circuit d'évacuation du digestat liquide et est relié à l'unité centrale 41 sous le niveau du digestat liquide 5 et, d'autre part, le circuit 14a est circuit d'évacuation du biogaz et est connecté à la partie supérieure de l'unité 41 dans une zone ne comportant pas de digestat liquide 5.

Le circuit 14a de biogaz appartient au réseau d'évacuation 35 de biogaz 25 et le circuit d'évacuation 14b de digestat liquide 5 appartient au réseau d'évacuation 34 de digestat liquide 5.

Comme indiqué précédemment, au cours de l'étape de digestion, un mélange de biogaz 5 et de digestat liquide 25 est extrait à travers l'élément de séparation perforé 13 et est acheminé dans la tuyauterie 14.

Le digestat liquide se retrouve alors acheminé principalement dans le circuit 14b vers la réserve en digestat liquide 5 de la cuve 42.

Le biogaz 5 quant à lui circule principalement dans le circuit 14a et traverse l'ouvrage 41, dans une zone ne comportant pas de digestat liquide 5, pour être acheminé, par le biais de la tuyauterie 44, vers le gazomètre 43.

De cette façon, l'unité 41 comporte, en surface du digestat liquide 25, une faible quantité de biogaz 5 provenant du circuit 14a reliant le digesteur 7 et l'unité 41.

La figure 11 représente schématiquement une vue en coupe d'une pluralité de digesteurs anaérobies de biomasse solide alimentés directement en digestat liquide par une unité de digestion complémentaire et de stockage de digestat liquide et de biogaz (liaison en parallèle), constitués d'une seule et même unité, au cours d'une étape de digestion dans des conditions anaérobies.

La figure 11 se concentre donc sur un système de production de biogaz comprenant une pluralité de digesteurs anaérobies de biomasse solide 100, 101, 102, 103 et 104 contenant de la biomasse solide directement alimentée en digestat liquide 5 en provenance d'une unité centrale de stockage de digestat liquide et de digestion complémentaire et une unité de stockage de biogaz constituant une seule et même unité, dénommé unité 1.

L'unité 1 est identique à l'unité décrite dans les figures 1 à 9, c'est-à-dire qu'elle comprend notamment un espace de stockage du biogaz 25 et une cuve remplie en digestat liquide 5.

Conformément à la figure 11, le système de production de biogaz peut comprendre une pluralité de digesteurs anaérobies entre le digesteur 100 et le digesteur 101.

Comme indiqué ci-avant, la figure 11 représente des digesteurs anaérobies de biomasse solide 100, 101, 102, 103 et 104 selon un mode de réalisation différent du digesteur anaérobie 7.

En particulier, chaque digesteur 100, 101, 102, 103 et 104 représenté sur la figure 11 est identique au digesteur anaérobie 7, représenté sur les figures 1 à 9, à l'exception du fait que la cuve 8 ne comporte pas de col 8c, ni d'épaulement 8b, au niveau de la partie supérieure de la cuve 8, et que l'élément séparation 13, situé en amont de l'ouverture 14 d'évacuation de digestat liquide et de biogaz, est fixée sur la surface supérieure de la cuve 8 et la paroi latérale contigüe à ladite surface.

Le digesteur 100 comprend une cuve 8, de forme essentiellement cylindrique, dotée d'un orifice d'entrée, ayant une surface inférieure ou égale à la surface totale de la partie supérieure de la cuve 8, sur laquelle un toit amovible 110 est apte à se refermer et/ou à s'ouvrir.

Plus précisément, le toit amovible 110 est apte à s'ouvrir et se refermer sur l'orifice d'entrée constituant la zone d'admission de la biomasse solide.

Sur la figure 11, le toit amovible 110 correspond à un autre mode de réalisation du toit amovible représenté dans les figures 1 à 10.

La cuve 8 présente au niveau de sa surface supérieure une tuyauterie 14, en saillie vers l'extérieur de la cuve, reliée à un circuit 14', qui s'étend verticalement et se divise en un circuit d'évacuation 14a de biogaz et un circuit d'évacuation 14b de digestat liquide.

De la même façon que précédemment, la tuyauterie 14 constitue une ouverture d'évacuation commune de digestat liquide et de biogaz.

Le circuit 14b permet d'évacuer du digestat liquide vers la cuve, remplie de digestat liquide 5, de l'unité 1 et le circuit d'évacuation 14a permet d'évacuer du biogaz vers l'espace de stockage rempli de biogaz 5 de l'unité 1.

D'une part, le circuit d'évacuation 14' est relié au circuit 14b par le biais d'une ramification, ayant de préférence la forme d'un coude arrondi, qui est piquée à la fois sur le circuit d'évacuation 14' et le circuit 14b d'évacuation de digestat liquide 5. En particulier, la ramification présente la forme d'un coude arrondi orienté vers le bas qui fait la jonction entre le circuit d'évacuation 14' et le circuit 14b d'évacuation de digestat liquide 5. La ramification appartient au circuit 14b.

La ramification peut présenter tout type de forme orientée vers le bas.

D'autre part, le circuit d'évacuation 14' est relié à son extrémité au circuit 14a d'évacuation de biogaz 25 par le biais d'une jonction à angle droit.

Le circuit 14a d'évacuation en biogaz 25 est positionné au-dessus du circuit 14b de digestat liquide 5 et est de préférence sensiblement parallèle à celui-ci.

En d'autres termes, le circuit d'évacuation 14' comprend deux types embranchements situés l'un au-dessus de l'autre, à savoir une ramification ayant la forme d'un coude arrondi et une jonction à angle droit, situé au-dessus de ladite ramification. Les deux embranchements donnent naissance aux circuits 14a et 14b de manière à relier chaque digesteur anaérobie 100, 101, 102, 103 et 104 respectivement à l'espace de stockage en biogaz 5 et la cuve remplie en digestat liquide 5 de l'unité 1.

En particulier, le circuit 14b est positionné de manière à permettre l'évacuation du digestat liquide directement dans la cuve de l'unité 1, c'est-à-dire à proximité du niveau du digestat liquide 5 au sein de la cuve de l'unité 1.

Parallèlement, le circuit 14a est positionné de manière à permettre l'évacuation du biogaz directement dans l'espace de stockage en biogaz de l'unité 1.

Le circuit d'évacuation 14b appartient à un réseau d'évacuation 34 en digestat liquide 5 et le circuit d'évacuation 14a en biogaz 25 appartient à un réseau d'évacuation 35 en biogaz 25.

Il en résulte que, d'une part, le réseau d'évacuation 34 de digestat liquide 5 comprend une pluralité de circuits d'évacuation de digestat liquide, notamment le circuit d'évacuation 14b de digestat liquide 5 et le circuit d'évacuation 14' de digestat liquide 5 et de biogaz 25 et, d'autre part, le réseau d'évacuation 35 de biogaz 25 comprend une pluralité de circuits d'évacuation de biogaz, notamment le circuit d'évacuation 14a de biogaz 25 et le circuit d'évacuation 14' de digestat liquide 5 et biogaz 25.

Autrement dit, le réseau d'évacuation 34 de digestat liquide 5 et le réseau d'évacuation 35 de biogaz 25 comprennent respectivement un circuit d'évacuation 14b en digestat liquide 5 et un circuit d'évacuation 14a en biogaz 5 qui sont distincts l'un de l'autre.

Chaque digesteur 100, 101, 102, 103 et 104 présente une conduite 14 et un circuit d'évacuation 14' de digestat liquide et de biogaz. Pour des raisons de simplicité, les références à la conduite 14 ainsi qu'aux circuits 14', 14a et 14b n'ont été représentées que pour le digesteur 100.

Chaque digesteur 100, 101, 102, 103 et 104 est alimenté en digestat liquide 5 par le biais respectivement d'un circuit d'alimentation 310, 410, 510, 610 et 710 en provenance de l'unité 1.

Les circuits d'alimentation 310, 410, 510, 610 et 710 en digestat liquide appartiennent à un réseau d'alimentation 33 en digestat liquide.

Comme indiqué ci-avant, l'ouverture d'alimentation en digestat liquide correspond également à une ouverture de vidange du digestat liquide.

L'étape de digestion est identique à l'étape de digestion décrite dans la figure 4.

L'alimentation en digestat liquide 5 s'effectue dans chaque digesteur 100, 101, 102 et 103 par le biais des circuits d'alimentation 310, 410, 510, 610 et 710 appartenant au réseau d'alimentation 33 en digestat liquide qui est relié à l'unité 1.

Les circuits d'alimentation 310, 410, 510, 610 et 710 sont munis respectivement d'une pompe d'alimentation 310b, 410b, 510b, 610b et 710b.

Au cours de l'étape de digestion de la biomasse solide, d'une part, le biogaz 25 est évacué dans le circuit d'évacuation 14' de digestat liquide et de biogaz ainsi que dans le circuit d'évacuation 14a vers l'espace de stockage en biogaz de l'unité 25.

D'autre part, le digestat liquide 5 est évacué dans le circuit d'évacuation 14' de digestat liquide et de biogaz ainsi que dans le circuit d'évacuation 14b vers la cuve remplie de digestat liquide 5 de l'unité 1.

Conformément à ce mode de réalisation, d'une part, le digestat liquide 5 et, d'autre part, le biogaz 25 sont directement évacués depuis chaque digesteur 100, 101, 102 et 103 vers l'unité 1.

Sur la figure 11 ont été représentés le sens de circulation 27 du digestat liquide dans le réseau d'alimentation 33 en digestat liquide, le sens de circulation 27" du mélange biogaz et digestat liquide dans le circuit 14', le sens de circulation 27' dans le réseau d'évacuation 35 du biogaz. En outre, le sens de circulation 27 dans la ramification du circuit 14b puis dans le réseau d'évacuation 34 du digestat liquide a été représenté.

La figure 11 représente un système de production de biogaz dans lequel une étape de digestion se déroule au même moment dans chaque digesteur 100, 101, 102, 103 et 104.

Conformément à une variante selon la présente invention, les étapes de digestion peuvent ne pas se dérouler de manière simultanée dans chaque digesteur anaérobie lorsque les digesteurs anaérobies sont directement alimentés en digestat liquide en provenance de l'unité 25.

Ainsi certains digesteurs peuvent être dans une phase de vidange anaérobie, ou de vidange aérobie ou de remplissage de digestat liquide, tandis que les autres digesteurs sont dans une phase de digestion anaérobie.

Il est à noter que, pour chaque digesteur anaérobie, les étapes de remplissage, de vidange aérobie et anaérobie sont identiques à celles précédemment décrites dans les figures 1 à 9.

La figure 12 représente schématiquement une vue en coupe d'une pluralité de digesteurs anaérobies de biomasse solide alimentés indirectement (liaison en série) en digestat liquide par une unité centrale de stockage de digestat liquide et de digestion complémentaire et d'une unité centrale de stockage de biogaz constitués d'une seule et même unité.

La figure 12 se concentre donc sur un système de production de biogaz comprenant une pluralité de digesteurs anaérobies 100, 101, 102, 103 et 104 contenant de la biomasse solide reliés en série à l'unité centrale de stockage de digestat liquide et de digestion complémentaire et une unité de stockage de biogaz constituant une seule et même unité, dénommé unité 1.

L'unité 1 est identique à l'unité décrite dans les figures 1 à 9, c'est-à-dire qu'elle comprend notamment un espace de stockage du biogaz 25 et une cuve remplie en digestat liquide 5.

Les digesteurs 100, 101, 102, 103 et 104 sont identiques à ceux décrits dans le mode réalisation illustré dans la figure 11.

L'étape de digestion se déroule de manière identique à l'étape de digestion décrite dans la figure 4.

Pour chaque digesteur anaérobie 101, 102, 103 et 104, l'alimentation en digestat liquide 5 s'effectue de façon indirecte depuis l'unité 1 car le digestat liquide 5 transite par le ou les digesteurs anaérobies situés directement ou non en amont de chaque digesteur anaérobie 101, 102 et 103.

Conformément au mode de réalisation décrit dans la figure 12, l'unité 1 alimente directement en digestat liquide 5 le digesteur anaérobie 100 duquel est évacué, au cours de l'étape de digestion, du digestat liquide 5 et du biogaz 25 par le biais du circuit d'évacuation 14' de digestat liquide et de biogaz.

Lors de la séparation du biogaz 25 et du digestat liquide 5 au niveau de l'entrée de la ramification 32', le digestat liquide 5 est acheminé dans le circuit d'évacuation 14b puis dans le circuit d'alimentation en direction du digesteur situé directement ou non en aval, par exemple le digesteur anaérobie 101.

Chaque circuit d'alimentation récupère le digestat liquide 5 ayant transité dans le digesteur anaérobie précédent.

Par ailleurs, les vannes 41, 41', 41" et 41'" montées sur le circuit d'évacuation 14b en digestat liquide sont en position fermée, notamment pour acheminer le digestat liquide vers le digesteur anaérobie suivant.

Conformément à une variante selon la présente invention, les étapes de digestion peuvent ne pas se dérouler de manière simultanée dans chaque digesteur anaérobie lorsque les digesteurs anaérobies sont indirectement alimentés en digestat liquide en provenance de l'unité 1.

Sur la figure 12 ont été représentés le sens de circulation 27 du digestat liquide dans le réseau d'alimentation 33 en digestat liquide, le sens de circulation 27" du mélange biogaz et digestat liquide dans le circuit 14', le sens de circulation 27' du biogaz dans le réseau d'évacuation 35 du biogaz. En outre, le sens de circulation 27 du digestat liquide dans la ramification 32' puis dans le circuit d'alimentation 310, 410, 510, 610 et 710 ainsi que dans le réseau d'évacuation 34 du digestat liquide ont été représentés ont été représentés.

Il est à noter que les étapes de remplissage, de vidange aérobie et anaérobie sont identiques à celles précédemment décrites dans les figures 1 à 9.

La figure 13 représente schématiquement une vue en coupe d'une pluralité de digesteurs anaérobies de biomasse solide alimentés à la fois directement ou indirectement en digestat liquide par une unité de digestion complémentaire et de stockage de digestat liquide et d'une unité centrale de biogaz (liaison en série et en parallèle) au cours d'une étape de digestion dans des conditions anaérobies.

La figure 13 se concentre sur un système de production de biogaz comprenant une pluralité de digesteurs anaérobies 100, 101, 102, 103 et 104 contenant de la biomasse solide.

Dans ce système, d'une part, les digesteurs anaérobies 100, 101 et 104 sont alimentés directement en digestat liquide 5 en provenance de l'unité 1, et, d'autre part, les digesteurs anaérobies 102 et 103 sont alimentés en digestat liquide 5 respectivement par les digesteurs 101 et 102.

En d'autres termes, les digesteurs anaérobies 100, 101 et 104 sont alimentés directement en digestat liquide 5 en provenance de l'unité 1.

L'unité 1 est identique à l'unité décrite dans les figures 1 à 9, c'est-à-dire qu'elle comprend notamment un espace de stockage du biogaz 25 et une cuve remplie en digestat liquide 5.

Les digesteurs 100, 101, 102, 103 et 104 sont identiques à ceux décrits dans le mode réalisation illustré dans la figure 11.

L'étape de digestion se déroule de manière identique à l'étape de digestion décrite à la fois dans les figures 4 et 7.

Sur la figure 13 ont été représentés le sens de circulation 27 du digestat liquide dans le réseau d'alimentation 33 en digestat liquide et dans le réseau d'évacuation 34 du digestat liquide ainsi que le sens de circulation 27' du biogaz dans le réseau d'évacuation 35 du biogaz.

Conformément à une variante selon la présente invention, les étapes de digestion peuvent ne pas se dérouler de manière simultanée dans chaque digesteur anaérobie.

En particulier, certains digesteurs peuvent être dans une phase de vidange anaérobie, ou de vidange aérobie ou de remplissage de digestat liquide, tandis que les autres digesteurs sont dans une phase de digestion anaérobie.

La figure 21 illustre une vue schématique de dessus d'un système de production de biogaz comprenant :
- une unité centrale de stockage de digestat liquide et de digestion complémentaire et une unité centrale de stockage de biogaz constituant une seule et même unité, dénommé unité 1,
- une pluralité de digesteurs anaérobies 50, 51, 52, 53, 54, 55 et 56 disposés autour de l'unité 1 formant ainsi un cercle autour de l'unité 1, chacun desdits digesteurs étant reliés à l'unité 1 par le biais d'un circuit d'alimentation en digestat liquide et un circuit d'évacuation en digestat liquide,
- une pluralité de digesteurs anaérobies de biomasse solide 60, 61, 62, 63, 64 et 65 qui sont chacun alimentés directement en digestat liquide 5 en provenance de l'unité 1 par le biais d'un réseau d'alimentation 33 en digestat liquide (liaison en parallèle),
- une pluralité de digesteurs anaérobies de biomasse solide 71, 72, 73, 74 et 75 qui sont chacun alimentés indirectement en digestat liquide 5 en provenance de l'unité 1 par le biais d'un réseau d'alimentation 33 en digestat liquide (liaison en série), et un digesteur anaérobie de biomasse solide 70 qui se trouve à la première place de la série (à l'extrémité ou en tête de la série) et qui est donc alimenté directement en digestat liquide 5 en provenance de l'unité 1 par le biais d'un réseau d'alimentation 33 en digestat liquide.

L'unité 1 est identique à l'unité décrite dans les figures précédentes.

L'unité 1 comprend une cuve, ayant une forme essentiellement cylindrique, remplie de digestat liquide, qui est surmontée d'une membrane étanche constituant un espace de stockage de gaz, lequel est rempli de biogaz.

Les digesteurs anaérobies 50, 51, 52, 53, 54, 55 et 56 sont disposés de manière à former un cercle autour de l'unité 1.

Chaque digesteur anaérobie 50, 51, 52, 53, 54, 55 et 56 peut correspondre au digesteur anaérobie 7 ou l'un des digesteurs 100 à 104 décrits précédemment.

Chaque digesteur anaérobie 50, 51, 52, 53, 54, 55 et 56 est relié à l'unité 1 par le biais d'un circuit d'alimentation en digestat liquide et d'un circuit d'évacuation de digestat liquide.

Sur la figure 14, les sens de circulation du digestat liquide entre chaque digesteur anaérobie 50, 51, 52, 53, 54, 55 et l'unité 1 ont été représentés.

Chaque digesteur anaérobie 60, 61, 62, 63, 64 et 65 peut correspondre au digesteur anaérobie 7 ou l'un des digesteurs 100 à 104 décrits précédemment.

Les digesteurs anaérobies 60, 61, 62, 63, 64 et 65 comprennent donc au moins une ouverture d'alimentation en digestat liquide, au moins une ouverture d'évacuation en digestat liquide, au moins une ouverture d'évacuation de biogaz, au moins une ouverture de vidange de digestat liquide et au moins une ouverture d'introduction et/ou d'évacuation d'air.

Pour des raisons de simplicité, seules les ouvertures d'alimentation en digestat liquide, les ouvertures d'évacuation de digestat liquide des digesteurs anaérobies 60, 61, 62, 63, 64 et 65 sont représentées.

Chaque digesteur anaérobie 60, 61, 62, 63, 64 et 65 est alimenté directement en digestat liquide en provenance de l'unité 1 par le biais d'un réseau d'alimentation 33 en digestat liquide (liaison en parallèle).

Plus précisément, chaque ouverture d'alimentation en digestat liquide est reliée à un circuit d'alimentation en digestat liquide appartenant au réseau d'alimentation 33 en digestat liquide.

Chaque digesteur anaérobie 60, 61, 62, 63, 64 et 65 est également relié à un circuit d'évacuation de digestat liquide appartenant à un réseau d'évacuation 34 de digestat liquide.

Les sens de circulation du digestat liquide entre l'unité 1 et chaque digesteur anaérobie 60, 61, 62, 63, 64 et 65 ont été représentés sur la figure 14.

Chaque digesteur anaérobie 70, 71, 72, 73, 74 et 75 peut correspondre au digesteur anaérobie 7 ou l'un des digesteurs 100 à 104 décrits précédemment.

Pour des raisons de simplicité, seules les ouvertures d'alimentation en digestat liquide, les ouvertures d'évacuation de digestat liquide des digesteurs anaérobies 70, 71, 72, 73, 74 et 75 sont représentées.

Chaque digesteur anaérobie 71, 72, 73, 74 et 75 est alimenté indirectement en digestat liquide en provenance de l'unité 1 par le biais d'un réseau d'alimentation 33 en digestat liquide (liaison en série).

En particulier, chaque digesteur anaérobie 71, 72, 73, 74 et 75 est alimenté en digestat liquide par le biais d'un circuit d'alimentation relié au digesteur anaérobie précédent c'est-à-dire que le digestat liquide 27 a transité dans le précédent digesteur anaérobie avant d'alimenter le suivant.

Chaque ouverture d'alimentation en digestat liquide est reliée à un circuit d'alimentation en digestat liquide appartenant au réseau d'alimentation 33 en digestat liquide.

Chaque digesteur anaérobie 70, 71, 72, 73, 74 et 75 est également relié à un circuit d'évacuation de digestat liquide appartenant à un réseau d'évacuation 34 de digestat liquide.

Les sens de circulation du digestat liquide entre l'unité 1 et chaque digesteur anaérobie 70, 71, 72, 73, 74 et 75 ont été représentés sur la figure 14.

Sur la figure 14 sont représentées des pompes de circulation du digestat liquide disposés sur les circuits d'alimentation en digestat liquide.

En outre, le système de production comporte un dispositif de chauffage du digestat liquide qui peut être placé à n'importe quel endroit du système. Le système de chauffage n'a pas été représenté sur la figure 14.

Par ailleurs, sur cette figure, pour des raisons de simplicité, le réseau d'évacuation de biogaz n'a pas été représenté.

Comme indiqué ci-avant chacun des digesteurs, représentés dans la figure 14, présente une ouverture d'évacuation de biogaz (non représenté), laquelle ouverture d'évacuation de biogaz peut être commune à l'ouverture d'évacuation de digestat liquide ou peut être séparée.

L'ouverture d'évacuation de biogaz est reliée à au moins un circuit d'évacuation de biogaz appartenant au réseau d'évacuation de biogaz.

Le système de production de biogaz, illustré dans la figure 14, présente donc une pluralité de digesteurs anaérobies de biomasse solide composés :
- de plusieurs digesteurs anaérobies de biomasse solide, alimentés directement en digestat liquide en provenance d'une unité centrale de stockage de digestat liquide et de digestion complémentaire et d'une unité centrale de stockage de biogaz constituant une seule et même unité (liaisons en parallèle), et
- de plusieurs digesteurs anaérobies de biomasse solide, alimentés directement en digestat liquide à travers un réseau d'alimentation 33 en provenance d'une unité centrale de stockage de digestat liquide et de digestion complémentaire et d'une unité centrale de stockage de biogaz constituant une seule et même unité (liaisons en parallèle), et
- de plusieurs digesteurs anaérobies de biomasse solide alimentés indirectement en digestat liquide en provenance d'une unité centrale de stockage de digestat liquide et de digestion complémentaire et d'une unité centrale de stockage de biogaz constituant une seule et même unité (liaisons en série).

Conformément à ce mode de réalisation, l'unité centrale de stockage de digestat liquide et de digestion complémentaire et l'unité centrale de stockage de biogaz sont constituées d'une seule et même unité.

En variante, l'unité centrale de stockage de digestat liquide et de digestion complémentaire et unité centrale de stockage de biogaz peuvent être deux unités distinctes comprenant chacune un ou plusieurs ouvrages.

Dans le système de production de biogaz tel qu'illustré, un ou plusieurs digesteurs anaérobies et l'unité 1 sont calorifugés.

## Revendications

1. Système de production de biogaz comprenant :
• au moins une unité centrale de stockage de digestat liquide et de digestion complémentaire (25, 41), constituée d'un ou plusieurs ouvrages, apte à contenir principalement du digestat liquide (5, 5'),
• au moins une unité centrale de stockage de biogaz (25, 43), constitué d'un ou plusieurs ouvrages, apte à contenir principalement du biogaz (25),
• une pluralité de digesteurs anaérobies dont certains sont des digesteurs de biomasse solide (7, 50, 51, 52, 53, 54, 55, 56, 60, 61, 62, 63, 64, 65, 70, 71, 72, 73, 74, 75, 100, 101, 102, 103, 104), chacun desdits digesteurs de biomasse solide (7, 50, 51, 52, 53, 54, 55, 56, 60, 61, 62, 63, 64, 65, 70, 71, 72, 73, 74, 75, 100, 101, 102, 103, 104) comprenant :
- une cuve (8) apte à contenir de la biomasse solide (23), comportant au moins une zone d'admission (9) de la biomasse solide (23), située sur la partie supérieure de la cuve, sur laquelle un toit (10, 110) est apte à s'ouvrir et à se refermer, et comportant en outre au moins une zone d'évacuation (9) de la biomasse solide digérée résiduelle, certaines desdites zones d'admission et d'évacuation de biomasse solide pouvant être communes, et
- au moins une ouverture d'alimentation (6b) en digestat liquide, située sur la partie inférieure du digesteur anaérobie (7, 50, 60, 70, 100), reliée à au moins un circuit d'alimentation (6'b, 310, 410, 510, 610, 710) de digestat liquide, apte(s) à permettre l'introduction de digestat liquide (5) dans ledit digesteur de biomasse solide (7, 50, 60, 70, 100), et
- au moins une ouverture d'évacuation (14) de digestat liquide, située sur la partie supérieure du digesteur anaérobie (7, 50, 60, 70, 100), reliée à au moins un circuit d'évacuation (14', 14b) de digestat liquide, apte à permettre l'évacuation de digestat liquide (5) dudit digesteur de biomasse solide (7, 50, 60, 70, 100),
- au moins une ouverture d'évacuation (14) de biogaz, située sur la partie supérieure du digesteur anaérobie (7, 50, 60, 70, 100), reliée à au moins un circuit d'évacuation (14', 14b) de biogaz, apte à permettre l'évacuation de biogaz (25) depuis ledit digesteur de biomasse solide (7, 50, 60, 70, 100), et
- au moins une ouverture de vidange (6b) de digestat liquide, située sur la partie inférieure du digesteur anaérobie (7, 50, 60, 70, 100), apte(s) à permettre la vidange de digestat liquide dudit digesteur de biomasse solide (7, 50, 60, 70, 100), et
- au moins une ouverture d'introduction et/ou d'évacuation d'air (16), située sur la partie supérieure du digesteur anaérobie (7, 50, 60, 70, 100), apte à permettre l'introduction et/ou l'évacuation d'air (24) dans ledit digesteur de biomasse solide (7, 50, 60, 70, 100),
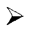 certaines ouvertures parmi lesdites ouvertures (6b, 14, 16) pouvant être communes, à l'exception des ouvertures d'alimentation (6b) et d'évacuation (14) de digestat liquide qui sont distinctes, et
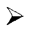 au moins un réseau d'alimentation (33) de digestat liquide, incluant une pluralité de circuits d'alimentation (6'b, 310, 410, 510, 610, 710) de digestat liquide, reliant certaines ouvertures d'alimentation (6b) de digestat liquide de certains digesteurs de biomasse solide (7, 50, 60, 70, 100) à l'unité centrale de stockage de digestat liquide et de digestion complémentaire (25, 41), apte à permettre l'alimentation de digestat liquide (5), par voie directe ou indirecte, dans lesdits digesteurs (7, 50, 60, 70, 100) depuis l'unité centrale de stockage de digestat liquide et de digestion complémentaire (25, 41), et
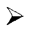 au moins un réseau d'évacuation (34) de digestat liquide, incluant une pluralité de circuits d'évacuation (14', 14b) de digestat liquide, reliant certaines ouvertures d'évacuation (14) de digestat liquide de certains digesteurs de biomasse solide (7, 50, 60, 70, 100) à l'unité centrale de stockage de digestat liquide et de digestion complémentaire (25, 41), apte à permettre l'évacuation de digestat liquide (5), par voie directe ou indirecte, depuis lesdits digesteurs (7, 50, 60, 70, 100) vers l'unité centrale de stockage de digestat liquide et de digestion complémentaire (25, 41), et
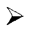 au moins un réseau d'évacuation (35) de biogaz, incluant une pluralité de circuits d'évacuation (14', 14a) de biogaz, reliant certaines ouvertures d'évacuation (14) de biogaz de certains digesteurs de biomasse solide (7, 50, 60, 70, 100) à l'unité centrale de stockage de biogaz (25, 43), apte à permettre l'évacuation de biogaz (25), par voie directe ou indirecte, depuis lesdits digesteurs (7, 50, 60, 70, 100) vers l'unité centrale de stockage de biogaz (25, 43),
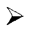 certaines parties desdits réseaux (33, 34, 35) pouvant être communes, et
• au moins un moyen de circulation (6a) de digestat liquide (5), de préférence une pompe, située sur le réseau d'alimentation (33) de digestat liquide (5) et/ou sur le réseau d'évacuation (34) de digestat liquide (5), apte à permettre la circulation du digestat liquide (5) à travers certains digesteurs de biomasse solide (7, 50, 60, 70, 100), et
• au moins un dispositif de chauffage (40) de digestat liquide, et
• dont, au moins un digesteur de biomasse solide (7, 50, 60, 70, 100) et au moins une unité centrale de stockage de digestat liquide et de digestion complémentaire sont calorifugées (25, 41).

2. Système de production de biogaz selon la revendication 1, **caractérisé en ce que** certains desdits digesteurs de biomasse solide (7, 50, 60, 70, 100) sont surmontés d'un moyen de préhension (20) apte à permettre l'alimentation de la biomasse solide (23).

3. Système selon la revendication 2, **caractérisé en ce que** le moyen de préhension (20) est un grappin comportant une pluralité de crochets (21) ou une benne comportant des godets.

4. Système selon l'une quelconque des revendications précédentes, **caractérisé en ce que**, dans certains desdits digesteurs de biomasse solide (7, 50, 60, 70, 100), au moins l'une des ouvertures d'évacuation (14) du digestat liquide comprend au moins un élément de séparation perforé (13) d'évacuation du digestat liquide (5), situé en amont de ladite ouverture d'évacuation (14) du digestat liquide (5), apte à séparer, d'une part, le digestat liquide (5) évacué et, d'autre part, la biomasse solide (23).

5. Système selon l'une quelconque des revendications précédentes, **caractérisé en ce que**, dans certains desdits digesteurs de biomasse solide (7, 50, 60, 70, 100), au moins l'une des ouvertures de vidange (6b) du digestat liquide (5) comprend au moins un élément de séparation perforé (18) de vidange du digestat liquide (5), situé en amont de ladite ouverture (6b) de vidange du digestat liquide (5), apte à séparer, d'une part, le digestat liquide (5) vidangé et, d'autre part, la biomasse solide (23).

6. Système selon la revendication 4 ou 5, **caractérisé en ce que** certains éléments de séparation perforés (13, 18) sont des plaques perforées.

7. Système selon la revendication 5, **caractérisé en ce que** l'élément de séparation perforé de vidange (18) du digestat liquide est un plancher perforé.

8. Système selon l'une quelconque des revendications précédentes, **caractérisé en ce que** sur certains desdits digesteur de biomasse solide (7, 50, 60, 70, 100) :
- la ou les ouverture(s) d'alimentation (6b) en digestat liquide, est (sont) munie(s) d'un moyen d'obstruction (30), de préférence une vanne, apte à obturer ou permettre le passage du digestat liquide (5) entre l'extérieur et l'intérieur du digesteur (7, 50, 60, 70, 100), et/ou
- la ou les ouverture(s) d'évacuation (14) de digestat liquide, est (sont) munie(s) d'un moyen d'obstruction (15), de préférence une vanne, apte à obturer ou permettre le passage du digestat liquide (5) entre l'intérieur et l'extérieur du digesteur (7, 50, 60, 70, 100), et/ou
- la ou les ouverture(s) d'évacuation (14) de biogaz, est (sont) munie(s) d'un moyen d'obstruction (15), de préférence une vanne, apte à obturer ou permettre le passage du biogaz (29) entre l'intérieur et l'extérieur du digesteur (7, 50, 60, 70, 100), et/ou
- la ou les ouverture(s) de vidange (6'b) de digestat liquide (5), est (sont) munie(s) d'un moyen d'obstruction (15), de préférence une vanne, apte à obturer ou permettre le passage du digestat liquide (5) entre l'intérieur et l'extérieur du digesteur (7, 50, 60, 70, 100), et/ou
- la ou les ouverture(s) d'introduction et/ou d'évacuation d'air (16), est (sont) munie(s) d'un moyen d'obstruction (17), de préférence une vanne, apte à obturer ou permettre le passage de l'air (24) entre l'intérieur et l'extérieur du digesteur (7, 50, 60, 70, 100).

9. Système selon l'une quelconque des revendications précédentes, **caractérisé en ce que** certains desdits digesteurs anaérobies de biomasse solide (7, 50, 60, 70, 100) comportent un joint liquide (11) positionné autour du toit (10) de la cuve (8), apte à empêcher la sortie du biogaz (25) contenu dans ledit digesteur (7, 50, 60, 70, 100) lorsque le toit (10) est en position fermée, et apte à empêcher les entrées d'air (24) dans ledit digesteur (7, 50, 60, 70, 100) lorsque le toit (10, 110) est en position fermée.

10. Système de production de biogaz selon l'une quelconque des revendications précédentes, **caractérisé en ce que** certains ouvrages composant l'unité centrale de stockage de digestat liquide et de digestion complémentaire (25, 41), d'une part, et certains ouvrages composant l'unité centrale de stockage de biogaz (25, 43), d'autre part, sont communs.

11. Système de production de biogaz selon l'une quelconque des revendications précédentes, **caractérisé en ce que** l'unité centrale de stockage de digestat liquide et de digestion complémentaire (25, 41), d'une part, et l'unité centrale de stockage de biogaz (25, 43), d'autre part, sont communes et constituent une seule et même unité centrale de de stockage de digestat liquide et de biogaz et de digestion complémentaire.

12. Système selon l'une quelconque des revendications précédentes, **caractérisé en ce que**, sur certains des digesteurs de biomasse solide (7, 50, 60, 70, 100), au moins une ouverture d'évacuation (14) de digestat liquide et une ouverture d'évacuation (14) de biogaz sont communes et sont reliées à au moins un circuit d'évacuation (14') d'un mélange de digestat liquide (5) et de biogaz (25), lequel circuit (14) est connecté à un moyen de séparation apte à séparer le biogaz (29) et le digestat liquide (5).

13. Procédé de production de biogaz comprenant au moins les étapes successives suivantes :
- une étape d'alimentation en biomasse solide (23), mise en œuvre par au moins un moyen de préhension (20), à travers une zone d'admission (9) située au niveau de la partie supérieure d'une cuve (8) d'au moins un digesteur de biomasse solide (7, 50, 51, 52, 53, 54, 55, 56, 60, 61, 62, 63, 64, 65, 70, 71, 72, 73, 74, 75, 100, 101, 102, 103, 104) calorifugé, tel que défini dans la revendication 1,
- une étape de remplissage de la cuve (8) dudit digesteur (7, 50, 60, 70, 100), avec du digestat liquide (5) pour immerger la biomasse solide (23),
- une étape de digestion, constituée d'une percolation du digestat liquide (5) à travers la biomasse solide (23) contenue dans ledit digesteur (7, 50, 60, 70, 100) et d'une diffusion du digestat liquide (5) dans ladite biomasse solide (23) dans des conditions anaérobies pour générer puis récupérer du biogaz (25), et
- une étape de vidange aérobie du digestat liquide (5) de la cuve (8) du digesteur, et
- une étape d'évacuation de la biomasse solide digérée résiduelle (23'), mise en œuvre par au moins un moyen de préhension (20), à travers une zone d'évacuation (9) de la biomasse solide du digesteur.

14. Procédé de production de biogaz selon la revendication 13, **caractérisé en ce qu'**il comprend en outre, entre l'étape de digestion et l'étape de vidange aérobie du digestat liquide (5), au moins les étapes suivantes :
- une étape de vidange anaérobie du digestat liquide (5) de la cuve (8) du digesteur (7, 50, 60, 70, 100), et
- une étape de re-remplissage de la cuve (8) du digesteur (7, 50, 60, 70, 100) avec un digestat liquide (5) après vidange anaérobie.

15. Procédé de production de biogaz, selon la revendication 14, **caractérisé en ce qu'**il comprend en outre, après l'étape de re-remplissage de la cuve (8) dudit digesteur (7, 50, 60, 70, 100) avec un digestat liquide (5), au moins une étape de digestion, constituée d'une percolation du digestat liquide (5) à travers la biomasse solide (23) et d'une diffusion du digestat liquide (5) dans la biomasse solide (23) pour générer puis récupérer le biogaz (25).

## Patentansprüche

1. System zur Herstellung von Biogas, das enthält:
• mindestens eine zentrale Einheit zum Speichern von flüssigem Gärrest und zur komplementären Gärung (25, 41), bestehend aus einem oder mehreren Bauteilen, die hauptsächlich flüssigen Gärrest (5, 5') enthalten kann,
• mindestens eine zentrale Einheit zum Speichern von Biogas (25, 43), bestehend aus einem oder mehreren Bauteilen, die hauptsächlich Biogas (25) enthalten kann,
• eine Vielzahl anaerober Fermenter, von denen einige Fermenter fester Biomasse (7, 50, 51, 52, 53, 54, 55, 56, 60, 61, 62, 63, 64, 65, 70, 71, 72, 73, 74, 75, 100, 101, 102, 103, 104) sind, wobei jeder der Fermenter fester Biomasse (7, 50, 51, 52, 53, 54, 55, 56, 60, 61, 62, 63, 64, 65, 70, 71, 72, 73, 74, 75, 100, 101, 102, 103, 104) enthält:
- einen Tank (8), der feste Biomasse (23) enthalten kann, der mindestens einen Einlassbereich (9) der festen Biomasse (23) aufweist, der sich am oberen Teil des Tanks befindet, auf dem ein Dach (10, 110) sich öffnen und schließen kann, und der weiter mindestens einen Abfuhrbereich (9) der restlichen vergärten festen Biomasse aufweist, wobei bestimmte der Einlass- und Abfuhrbereiche fester Biomasse gemeinsam sein können, und
- mindestens eine Zufuhröffnung (6b) von flüssigem Gärrest, die sich im unteren Teil des anaeroben Fermenters (7, 50, 60, 70, 100) befindet, verbunden mit mindestens einer Zufuhrleitung (6'b, 310, 410, 510, 610, 710) von flüssigem Gärrest, die die Einführung von flüssigem Gärrest (5) in den Fermenter fester Biomasse (7, 50, 60, 70, 100) erlauben kann(können), und
- mindestens eine Abfuhröffnung (14) von flüssigem Gärrest, die sich im oberen Teil des anaeroben Fermenters (7, 50, 60, 70, 100) befindet, verbunden mit mindestens einer Abfuhrleitung (14', 14b) von flüssigem Gärrest, die die Abfuhr von flüssigem Gärrest (5) aus dem Fermenter fester Biomasse (7, 50, 60, 70, 100) erlauben kann, und
- mindestens eine Abfuhröffnung (14) von Biogas, die sich im oberen Teil des anaeroben Fermenters (7, 50, 60, 70, 100) befindet, verbunden mit mindestens einer Abfuhrleitung (14', 14b) von Biogas, die die Abfuhr von Biogas (25) vom Fermenter fester Biomasse (7, 50, 60, 70, 100) erlauben kann, und
- mindestens eine Ablassöffnung (6b) von flüssigem Gärrest, die sich im unteren Bereich des anaeroben Fermenters (7, 50, 60, 70, 100) befindet, die das Ablassen von flüssigem Gärrest aus dem Fermenter fester Biomasse (7, 50, 60, 70, 100) erlauben kann(können), und
- mindestens eine Lufteinführ- und/oder Abfuhröffnung (16), die sich im oberen Teil des anaeroben Fermenters (7, 50, 60, 70, 100) befindet, die die Einführung und/oder die Abfuhr von Luft (24) im Fermenter fester Biomasse (7, 50, 60, 70, 100) erlauben kann,
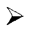 wobei bestimmte Öffnungen unter den Öffnungen (6b, 14, 16) gemeinsam sein können, mit Ausnahme der Zufuhr- (6b) und Abfuhröffnungen (14) von flüssigem Gärrest, die getrennt sind, und
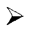 mindestens ein Zufuhrnetz (33) von flüssigem Gärrest, das eine Vielzahl von Zufuhrleitungen (6'b, 310, 410, 510, 610, 710) von flüssigem Gärrest enthält, das bestimmte Zufuhröffnungen (6b) von flüssigem Gärrest bestimmter Fermenter fester Biomasse (7, 50, 60, 70, 100) mit der zentralen Einheit zum Speichern von flüssigem Gärrest und zur komplementären Vergärung (25, 41) verbindet, das die Zufuhr von flüssigem Gärrest (5) auf direktem oder indirektem Weg in die Fermenter (7, 50, 60, 70, 100) von der zentralen Einheit zum Speichern von flüssigem Gärrest und zur komplementären Vergärung (25, 41) erlauben kann, und
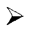 mindestens ein Abfuhrnetz (34) von flüssigem Gärrest, das eine Vielzahl von Abfuhrleitungen (14', 14b) von flüssigem Gärrest enthält, das bestimmte Abfuhröffnungen (14) von flüssigem Gärrest bestimmter Fermenter fester Biomasse (7, 50, 60, 70, 100) mit der zentralen Einheit zum Speichern von flüssigem Gärrest und zur komplementären Vergärung (25, 41) verbindet, das die Abfuhr von flüssigem Gärrest (5) auf direktem oder indirektem Weg von den Fermentern (7, 50, 60, 70, 100) zur zentralen Einheit zum Speichern von flüssigem Gärrest und zur komplementären Vergärung (25, 41) erlauben kann, und
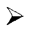 mindestens ein Abfuhrnetz (35) von Biogas, das eine Vielzahl von Abfuhrleitungen (14', 14a) von Biogas enthält, das bestimmte Abfuhröffnungen (14) von Biogas bestimmter Fermenter fester Biomasse (7, 50, 60, 70, 100) mit der zentralen Einheit zum Speichern von Biogas (25, 43) verbindet, das die Abfuhr von Biogas (25) auf direktem oder indirektem Weg von den Fermentern (7, 50, 60, 70, 100) zur zentralen Einheit zum Speichern von Biogas (25, 43) erlauben kann,
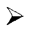 wobei bestimmte Teile der Netze (33, 34, 35) gemeinsam sein können, und
• mindestens eine Umwälzeinrichtung (6a) von flüssigem Gärrest (5), vorzugsweise eine Pumpe, die sich auf dem Zufuhrnetz (33) von flüssigem Gärrest (5) und/oder auf dem Abfuhrnetz (34) von flüssigem Gärrest (5) befindet, die die Umwälzung des flüssigen Gärrests (5) durch bestimmte Fermenter fester Biomasse (7, 50, 60, 70, 100) hindurch erlauben kann, und
• mindestens eine Heizvorrichtung (40) von flüssigem Gärrest, und
• wovon mindestens ein Fermenter fester Biomasse (7, 50, 60, 70, 100) und mindestens eine zentrale Einheit zum Speichern von flüssigem Gärrest und zur komplementären Vergärung (25, 41) wärmeisoliert sind.

2. System zur Herstellung von Biogas nach Anspruch 1, **dadurch gekennzeichnet, dass** bestimmte der Fermenter fester Biomasse (7, 50, 60, 70, 100) von einer Greifeinrichtung (20) überlagert werden, die die Zufuhr der festen Biomasse (23) erlauben kann.

3. System nach Anspruch 2, **dadurch gekennzeichnet, dass** die Greifeinrichtung (20) ein Greifer, der eine Vielzahl von Haken (21) aufweist, oder ein Becher aufweisender Kübel ist.

4. System nach einem der vorhergehenden Ansprüche, **dadurch gekennzeichnet, dass** bei bestimmten der Fermenter fester Biomasse (7, 50, 60, 70, 100) mindestens eine der Abfuhröffnungen (14) des flüssigen Gärrests mindestens ein gelochtes Trennelement (13) zur Abfuhr des flüssigen Gärrests (5) enthält, das sich stromaufwärts vor der Abfuhröffnung (14) des flüssigen Gärrests (5) befindet, das einerseits das abgeführte flüssige Gärrest (5) und andererseits die feste Biomasse (23) trennen kann.

5. System nach einem der vorhergehenden Ansprüche, **dadurch gekennzeichnet, dass** bei bestimmten der Fermenter fester Biomasse (7, 50, 60, 70, 100) mindestens eine der Ablassöffnungen (6b) des flüssigen Gärrests (5) mindestens ein gelochtes Trennelement (18) zum Ablassen des flüssigen Gärrests (5) enthält, das sich stromaufwärts vor der Ablassöffnung (6b) des flüssigen Gärrests (5) befindet, das einerseits den abgelassenen flüssigen Gärrest (5) und andererseits die feste Biomasse (23) trennen kann.

6. System nach Anspruch 4 oder 5, **dadurch gekennzeichnet, dass** bestimmte der gelochten Trennelemente (13, 18) Lochplatten sind.

7. System nach Anspruch 5, **dadurch gekennzeichnet, dass** das gelochte Ablass-Trennelement (18) des flüssigen Gärrests ein Lochboden ist.

8. System nach einem der vorhergehenden Ansprüche, **dadurch gekennzeichnet, dass** bei bestimmten der Fermenter fester Biomasse (7, 50, 60, 70, 100):
- die Zufuhröffnung(en) (6b) von flüssigem Gärrest mit einer Verschlusseinrichtung (30), vorzugsweise einem Ventil, versehen ist(sind), die den Durchgang des flüssigen Gärrests (5) zwischen der Innen- und Außenseite des Fermenters (7, 50, 60, 70, 100) verschließen oder erlauben kann, und/oder
- die Abfuhröffnung(en) (14) von flüssigem Gärrest mit einer Verschlusseinrichtung (15), vorzugsweise einem Ventil, versehen ist(sind), die den Durchgang des flüssigen Gärrests (5) zwischen der Innen- und Außenseite des Fermenters (7, 50, 60, 70, 100) verschließen oder erlauben kann, und/oder
- die Abfuhröffnung (en) (14) von Biogas mit einer Verschlusseinrichtung (15), vorzugsweise einem Ventil, versehen ist (sind), die den Durchgang des Biogases (29) zwischen der Innen- und Außenseite des Fermenters (7, 50, 60, 70, 100) verschließen oder erlauben kann, und/oder
- die Ablassöffnung(en) (6'b) von flüssigem Gärrest (5) mit einer Verschlusseinrichtung (15), vorzugsweise einem Ventil, versehen ist(sind), die den Durchgang des flüssigen Gärrests (5) zwischen der Innen- und Außenseite des Fermenters (7, 50, 60, 70, 100) verschließen oder erlauben kann, und/oder
- die Lufteinführ- und/oder Abfuhröffnung(en) (16) mit einer Verschlusseinrichtung (17), vorzugsweise einem Ventil, versehen sind, die den Durchgang der Luft (24) zwischen der Innen- und Außenseite des Fermenters (7, 50, 60, 70, 100) verschließen oder erlauben kann.

9. System nach einem der vorhergehenden Ansprüche, **dadurch gekennzeichnet, dass** bestimmte der anaeroben Fermenter fester Biomasse (7, 50, 60, 70, 100) eine Flüssigdichtung (11) aufweisen, die um das Dach (10) des Tanks (8) herum positioniert ist, die den Austritt des im Fermenter (7, 50, 60, 70, 100) enthaltenen Biogases (25) verhindern kann, wenn das Dach (10) in geschlossener Stellung ist, und die die Eintritte von Luft (24) in den Fermenter (7, 50, 60, 70, 100) verhindern kann, wenn das Dach (10, 110) in geschlossener Stellung ist.

10. System zur Herstellung von Biogas nach einem der vorhergehenden Ansprüche, **dadurch gekennzeichnet, dass** bestimmte die zentrale Einheit zum Speichern von flüssigem Gärrest und komplementärer Vergärung (25, 41) bildende Bauteile einerseits und bestimmte die zentrale Einheit zum Speichern von Biogas (25, 43) bildende Bauteile andererseits gemeinsam sind.

11. System zur Herstellung von Biogas nach einem der vorhergehenden Ansprüche, **dadurch gekennzeichnet, dass** die zentrale Einheit zum Speichern von flüssigem Gärrest und zur komplementären Vergärung (25, 41) einerseits und die zentrale Einheit zum Speichern von Biogas (25, 43) andererseits gemeinsam sind und eine einzige zentrale Einheit zum Speichern von flüssigem Gärrest und von Biogas und zur komplementären Vergärung bilden.

12. System nach einem der vorhergehenden Ansprüche, **dadurch gekennzeichnet, dass** in bestimmten der Fermenter fester Biomasse (7, 50, 60, 70, 100) mindestens eine Abfuhröffnung (14) von flüssigem Gärrest und eine Abfuhröffnung (14) von Biogas gemeinsam sind und mit mindestens einer Abfuhrleitung (14') einer Mischung von flüssigem Gärrest (5) und von Biogas (25) verbunden sind, wobei die Leitung (14) mit einer Trenneinrichtung verbunden ist, die das Biogas (29) und den flüssigen Gärrest (5) trennen kann.

13. Verfahren zur Erzeugung von Biogas, das mindestens die folgenden aufeinanderfolgenden Schritte enthält:
- einen Schritt der Zufuhr fester Biomasse (23), der von mindestens einer Greifeinrichtung (20) mittels eines Einlassbereichs (9) durchgeführt wird, der sich im Bereich des oberen Teils eines Tanks (8) mindestens eines wärmeisolierten Fermenters fester Biomasse (7, 50, 51, 52, 53, 54, 55, 56, 60, 61, 62, 63, 64, 65, 70, 71, 72, 73, 74, 75, 100, 101, 102, 103, 104) befindet, wie in Anspruch 1 definiert,
- einen Schritt des Füllens des Tanks (8) des Fermenters (7, 50, 60, 70, 100) mit flüssigem Gärrest (5), um die feste Biomasse (23) einzutauchen,
- einen Schritt der Vergärung, bestehend aus einer Durchsickerung des flüssigen Gärrests (5) durch die im Fermenter (7, 50, 60, 70, 100) enthaltene feste Biomasse (23) und einer Ausbreitung des flüssigen Gärrests (5) in der festen Biomasse (23) unter anaeroben Bedingungen, um Biogas (25) zu erzeugen und wiederzugewinnen, und
- einen Schritt des aeroben Ablassens des flüssigen Gärrests (5) aus dem Tank (8) des Fermenters, und
- einen Schritt des Abführens der restlichen vergärten festen Biomasse (23'), durchgeführt von mindestens einer Greifeinrichtung (20), durch einen Abfuhrbereich (9) der festen Biomasse aus dem Fermenter hindurch.

14. Verfahren zur Erzeugung von Biogas nach Anspruch 13, **dadurch gekennzeichnet, dass** es außerdem zwischen dem Schritt der Vergärung und dem Schritt des aeroben Ablassens des flüssigen Gärrests (5) mindestens die folgenden Schritte enthält:
- einen Schritt des anaeroben Ablassens des flüssigen Gärrests (5) aus dem Tank (8) des Fermenters (7, 50, 60, 70, 100), und
- einen Schritt der erneuten Befüllung des Tanks (8) des Fermenters (7, 50, 60, 70, 100) mit einem flüssigen Gärrest (5) nach anaerobem Ablassen.

15. Verfahren zur Erzeugung von Biogas nach Anspruch 14, **dadurch gekennzeichnet, dass** es außerdem nach dem Schritt der erneuten Befüllung des Tanks (8) des Fermenters (7, 50, 60, 70, 100) mit einem flüssigen Gärrest (5) mindestens einen Vergärungsschritt enthält, der aus einer Durchsickerung des flüssigen Gärrests (5) durch die feste Biomasse (23) hindurch und einer Ausbreitung des flüssigen Gärrests (5) in der festen Biomasse (23) besteht, um das Biogas (25) zu erzeugen und wiederzugewinnen.

## Claims

1. Biogas production system, comprising:
• at least one central unit for storing liquid digestate and for additional digestion (25, 41), consisting of one or more works, which are capable of mainly containing liquid digestate (5, 5'),
• at least one central unit for storing biogas (25, 43), consisting of one or more works, which are capable of mainly containing biogas (25),
• a plurality of anaerobic digestors, some of which are solid biomass digestors (7, 50, 51, 52, 53, 54, 55, 56, 60, 61, 62, 63, 64, 65, 70, 71, 72, 73, 74, 75, 100, 101, 102, 103, 104), each of said solid biomass digestors (7, 50, 51, 52, 53, 54, 55, 56, 60, 61, 62, 63, 64, 65, 70, 71, 72, 73, 74, 75, 100, 101, 102, 103, 104) comprising:
- a tank (8) capable of containing solid biomass (23), including at least one zone (9) for introducing solid biomass (23), located on the upper part of the tank, on which a roof (10, 110) is capable of opening and closing, and also including at least one zone (9) for removing the residual digested solid biomass, some of said zones for introducing and removing solid biomass possibly being common, and
- at least one aperture (6b) for feeding in liquid digestate, located on the lower part of the anaerobic digestor (7, 50, 60, 70, 100), connected to at least one circuit (6'b, 310, 410, 510, 610, 710) for feeding in liquid digestate, which is (are) capable of allowing the introduction of liquid digestate (5) into said solid biomass digestor (7, 50, 60, 70, 100), and
- at least one aperture (14) for removing liquid digestate, located on the upper part of the anaerobic digestor (7, 50, 60, 70, 100), connected to at least one circuit (14', 14b) for removing liquid digestate, which is capable of allowing the removal of liquid digestate (5) from said solid biomass digestor (7, 50, 60, 70, 100),
- at least one aperture (14) for removing biogas, located on the upper part of the anaerobic digestor (7, 50, 60, 70, 100), connected to at least one circuit (14', 14b) for removing biogas, which is capable of allowing the removal of biogas (25) from said solid biomass digestor (7, 50, 60, 70, 100), and
- at least one aperture (6b) for discharging liquid digestate, located on the lower part of the anaerobic digestor (7, 50, 60, 70, 100), which is (are) capable of allowing the discharging of liquid digestate from said solid biomass digestor (7, 50, 60, 70, 100), and
- at least one aperture (16) for introducing and/or removing air, located on the upper part of the anaerobic digestor (7, 50, 60, 70, 100), which is capable of allowing the introduction and/or removal of air (24) in said solid biomass digestor (7, 50, 60, 70, 100),
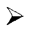 certain apertures among said apertures (6b, 14, 16) possibly being common, with the exception of the apertures for feeding in (6b) and for removing (14) liquid digestate, which are distinct, and
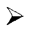 at least one network (33) for introducing liquid digestate, including a plurality of circuits (6'b, 310, 410, 510, 610, 710) for feeding in liquid digestate, connecting certain apertures (6b) for feeding liquid digestate from certain solid biomass digestors (7, 50, 60, 70, 100) to the central unit for storing liquid digestate and for additional digestion (25, 41), which is capable of allowing the feeding of liquid digestate (5), via a direct or indirect route, into said digestors (7, 50, 60, 70, 100) from the central unit for storing liquid digestate and for additional digestion (25, 41), and
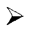 at least one network (34) for removing liquid digestate, including a plurality of circuits (14', 14b) for removing liquid digestate, connecting certain apertures (14) for removing liquid digestate from certain solid biomass digestors (7, 50, 60, 70, 100) to the central unit for storing liquid digestate and for additional digestion (25, 41), which is capable of allowing the removal of liquid digestate (5), via a direct or indirect route, from said digestors (7, 50, 60, 70, 100) to the central unit for storing liquid digestate and for additional digestion (25, 41), and
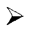 at least one network (35) for removing biogas, including a plurality of circuits (14', 14a) for removing biogas, connecting certain apertures (14) for removing biogas from certain solid biomass digestors (7, 50, 60, 70, 100) to the central unit for storing biogas (25, 43), which is capable of allowing the removal of biogas (25), via a direct or indirect route, from said digestors (7, 50, 60, 70, 100) to the central unit for storing biogas (25, 43),
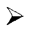 certain parts (33, 34, 35) of said networks possibly being common, and
• at least one means (6a) for circulating liquid digestate (5), preferably a pump, located on the network (33) for feeding liquid digestate (5) and/or on the network (34) for removing liquid digestate (5), which is capable of allowing the circulation of the liquid digestate (5) through certain solid biomass digestors (7, 50, 60, 70, 100), and
• at least one device (40) for heating liquid digestate, and
• of which at least one solid biomass digestor (7, 50, 60, 70, 100) and at least one central unit for storing liquid digestate and for additional digestion are thermally insulated (25, 41).

2. Biogas production system according to Claim 1, **characterized in that** some of said solid biomass digestors (7, 50, 60, 70, 100) have mounted on them a handling means (20) which is capable of allowing the feeding of the solid biomass (23).

3. Biogas production system according to Claim 2, **characterized in that** the handling means (20) is a grappler including a plurality of hooks (21) or a tub including buckets.

4. Biogas production system according to any one of the preceding claims, **characterized in that**, in some of said solid biomass digestors (7, 50, 60, 70, 100), at least one of the apertures (14) for removing liquid digestate comprises at least one perforated separation member (13) for removing liquid digestate (5), located upstream of said aperture (14) for removing liquid digestate (5), which is capable of separating, on the one hand, the removed liquid digestate (5) and, on the other hand, the solid biomass (23).

5. Biogas production system according to any one of the preceding claims, **characterized in that**, in some of said solid biomass digestors (7, 50, 60, 70, 100), at least one of the apertures (6b) for discharging liquid digestate (5) comprises at least one perforated separation member (18) for discharging liquid digestate (5), located upstream of said aperture (6b) for discharging liquid digestate (5), which is capable of separating, on the one hand, the discharged liquid digestate (5) and, on the other hand, the solid biomass (23) .

6. Biogas production system according to Claim 4 or 5, **characterized in that** certain perforated separation members (13, 18) are perforated plates.

7. Biogas production system according to Claim 5, **characterized in that** the perforated separation member (18) for discharging liquid digestate is a perforated floor.

8. Biogas production system according to any one of the preceding claims, **characterized in that**, on some of said solid biomass digestors (7, 50, 60, 70, 100):
- the aperture(s) (6b) for feeding liquid digestate are equipped with an obstruction means (30), preferably a valve, which is capable of closing off or of allowing the passage of the liquid digestate (5) between the exterior and the interior of the digestor (7, 50, 60, 70, 100), and/or
- the aperture(s) (14) for removing liquid digestate are equipped with an obstruction means (15), preferably a valve, which is capable of closing off or of allowing the passage of the liquid digestate (5) between the interior and the exterior of the digestor (7, 50, 60, 70, 100), and/or
- the aperture(s) (14) for removing biogas are equipped with an obstruction means (15), preferably a valve, which is capable of closing off or of allowing the passage of the biogas (29) between the interior and the exterior of the digestor (7, 50, 60, 70, 100), and/or
- the aperture(s) (6'b) for discharging liquid digestate (5) are equipped with an obstruction means (15), preferably a valve, which is capable of closing off or of allowing the passage of the liquid digestate (5) between the interior and the exterior of the digestor (7, 50, 60, 70, 100), and/or
- the aperture(s) (16) for introducing and/or removing air are equipped with an obstruction means (17), preferably a valve, which is capable of closing off or of allowing the passage of air (24) between the interior and the exterior of the digestor (7, 50, 60, 70, 100).

9. Biogas production system according to any one of the preceding claims, **characterized in that** some of said solid biomass anaerobic digestors (7, 50, 60, 70, 100) include a liquid seal (11) positioned around the roof (10) of the tank (8), which is capable of preventing the exit of the biogas (25) contained in said digestor (7, 50, 60, 70, 100) when the roof (10) is in the closed position, and capable of preventing the entry of air (24) into said digestor (7, 50, 60, 70, 100) when the roof (10, 110) is in the closed position.

10. Biogas production system according to any one of the preceding claims, **characterized in that** certain works of which the central unit for storing liquid digestate and for additional digestion (25, 41) are composed, on the one hand, and certain works of which the central unit for storing biogas (25, 43) are composed, on the other hand, are common.

11. Biogas production system according to any one of the preceding claims, **characterized in that** the central unit for storing liquid digestate and for additional digestion (25, 41), on the one hand, and the central unit for storing biogas (25, 43), on the other hand, are common and constitute one and the same central unit for storing liquid digestate and biogas and for additional digestion.

12. Biogas production system according to any one of the preceding claims, **characterized in that**, on some of the solid biomass digestors (7, 50, 60, 70, 100), at least one aperture (14) for removing liquid digestate and one aperture (14) for removing biogas are common and are connected to at least one circuit (14') for removing a mixture of liquid digestate (5) and of biogas (25), which circuit (14) is connected to a separation means that is capable of separating the biogas (29) and the liquid digestate (5) .

13. Biogas production process comprising at least the following successive steps:
- a step of feeding solid biomass (23), performed by at least one handling means (20), through an introduction zone (9) located in the upper part of a tank (8) of at least one thermally insulated solid biomass digestor (7, 50, 51, 52, 53, 54, 55, 56, 60, 61, 62, 63, 64, 65, 70, 71, 72, 73, 74, 75, 100, 101, 102, 103, 104), as defined in Claim 1,
- a step of filling the tank (8) of said digestor (7, 50, 60, 70, 100) with liquid digestate (5) to immerse the solid biomass (23),
- a step of digestion, consisting of percolation of the liquid digestate (5) through the solid biomass (23) contained in said digestor (7, 50, 60, 70, 100) and of diffusion of the liquid digestate (5) into said solid biomass (23) under anaerobic conditions to generate and then recover biogas (25), and
- a step of aerobic discharging of the liquid digestate (5) from the tank (8) of the digestor, and
- a step of removing the residual digested solid biomass (23'), performed by at least one handling means (20), through a zone (9) for removing the solid biomass from the digestor.

14. Biogas production process according to Claim 13, **characterized in that** it also comprises, between the digestion step and the step of aerobic discharging of the liquid digestate (5), at least the following steps:
- a step of anaerobic discharging of the liquid digestate (5) from the tank (8) of the digestor (7, 50, 60, 70, 100), and
- a step of refilling the tank (8) of the digestor (7, 50, 60, 70, 100) with a liquid digestate (5) after anaerobic discharging.

15. Biogas production process according to Claim 14, **characterized in that** it also comprises, after the step of refilling the tank (8) of said digestor (7, 50, 60, 70, 100) with a liquid digestate (5), at least one digestion step, consisting of percolation of the liquid digestate (5) through the solid biomass (23) and diffusion of the liquid digestate (5) in the solid biomass (23) to generate and then recover the biogas (25) .
